# EUROPEAN PATENT APPLICATION

(11) **EP 4 497 457 A1**
(43) Date of publication of application: **29.01.2025**
(21) Application number: 23188498.2
(22) Date of filing: 28.07.2023
(51) Int. Cl.: A61M 5/315

(54) **DOSING MECHANISM**

(71) Applicant: medmix Switzerland AG, 9469 Haag (Rheintal) (CH)
(72) Inventor: KOEHLER, Timm, 9469 Haag (Rheintal) (CH); BOOSEY, Paul, 9469 Haag (Rheintal) (CH); KEITEL, Joachim, 9469 Haag (Rheintal) (CH); MURKUTE, Rahul, 9469 Haag (Rheintal) (CH)
(74) Representative: Manitz Finsterwald Patent- und Rechtsanwaltspartnerschaft mbB

(57) **Abstract**

A dosing mechanism comprises a first member, a second member and a friction brake located in between a first contact section of the first member and a second contact section of the second member and the first member is configured to rotate with respect to the second member during setting of a dose. The first member is configured to transfer an axial force to the second member in a proximal direction during dose delivery. The first contact section is configured to press the friction brake towards the second contact section during dose delivery so that the friction brake provides a rotation brake between the first and second member. A hard stop acts between the first member and the second member during dose delivery, wherein the hard stop is configured to restrict a movement of the first contact section towards the second contact section during dose delivery.

## Description

### FIELD

The present disclosure relates to dosing mechanisms for a medicament delivery device and to medicament delivery devices comprising the dosing mechanisms.

### BACKGROUND

Dosing mechanisms for medicament delivery devices are configured to deliver at least one predetermined dose of medicament to a patient. Usually, such dosing mechanisms allowed to first set the dose to be delivered in a dose setting state of the dosing mechanism and to then deliver a set dose in a dose delivery state of the dosing mechanism.

For dose setting, dosing mechanisms usually comprise a dose setting element that is actuated by a user to set the dose. For example, the dose setting element may be configured to be rotated by the user to set the dose. The dosing mechanism thereby may be configured to allow only a single predetermined dose to be set or several varying predetermined doses. Furthermore, dosing mechanisms usually comprise an actuation element, such as a push button, that is configured to be actuated by the user to deliver a set dose.

To avoid delivery of the medicament during dose setting and to allow the delivery of the medicament during dose delivery, the dosing mechanisms usually comprise one or more members that are configured to move with respect to at least one other member during dose setting and that are restricted from moving with respect to the at least one other member during dose delivery or vice versa. This allows to transfer a force to a delivery member, such as a piston rod, only during dose delivery but not during dose setting.

One such dosing mechanism is exemplarily described in document US20060258988A1. The dosing mechanism thereby comprises a piston rod and a threaded part that is threadedly engaged with the piston rod. During dose setting, the threaded part is configured to rotate with respect to the piston rod and thereby to advance in an axial direction with respect to the piston rod to set a dose. During dose delivery, the threaded part is configured to remain rotationally stationary with respect to the piston rod. Axial movement of the threaded part then pushes the piston rod proximally to deliver a set dose. A rotation of the threaded part is thereby prevented by a flexible detent member that engages with axial splines provided at a housing of the device. If, however, the flexible detent member is not strong enough to prevent rotation of the threaded part, the threaded part could rotate along the thread to the piston rod instead of pushing the piston rod via the locked thread. An amount of medicament delivered then would be lower than the dose previously set.

As it can exemplarily be deduced from the device described in document US20060258988A1, any relative movement of members during dose delivery that are configured to remain stationary with respect to each other during dose delivery may compromise dose accuracy. At the same time, any inhibition or restriction of relative movement of such members during dose setting might impede or prohibit setting of a dose.

Accordingly, there is a need to reliably restrict movement between two members of a dosing mechanism during dose delivery while at the same time allowing movement between these two members during dose setting.

In a further aspect, medicament delivery devices usually comprise a medicament holder that contains the medicament to be delivered. With reusable devices, the medicament holder usually is releasably attached to a housing of the medicament delivery device to allow changing the medicament holder or a cartridge provided within the medicament holder after having delivered a last dose of medicament. Such medicament delivery devices further usually comprise a cap that covers the medicament holder when the medicament delivery device is not in use. Detachment of the cap prior to using the medicament delivery device then may result in a loosening or even detachment of the medicament holder if the cap touches our engages the medicament holder, for example by a form fit or a frictional coupling.

Accordingly, there is a need to reliably prevent detachment of a medicament holder from a medicament delivery device upon removing a cap from the device.

In yet another aspect, medicament delivery devices having a housing and a cap attachable to the housing may require to attach the cap to the housing in a predetermined rotational orientation, for example to align visual indicators or other features provided at the cap and the housing. Since both the cap and the housing usually are configured as essentially cylindrical bodies, they in principle also allow to attach the cap to the housing in arbitrary rotational orientations.

Accordingly, there is a need to provide a medicament delivery device that ensures detachment of a cap to a housing of the device in a predetermined rotational orientation.

### SUMMARY

The present disclosure provides a dosing mechanism for a medicament delivery device according to the independent claims. Embodiments are given in the dependent claims, the description, and the drawings.

In one aspect, the present disclosure is directed at a dosing mechanism for a medicament delivery device, the dosing mechanism comprising a first member, a second member and a friction brake. The friction brake thereby is located in between a first contact section of the first member and a second contact section of the second member and the first member is configured to rotate with respect to the second member around a longitudinal axis during setting of a dose of medicament to be delivered. Furthermore, the first member is configured to transfer an axial force to the second member in a proximal direction parallel to the longitudinal axis during dose delivery to deliver the dose. The first contact section is configured to press the friction brake towards the second contact section during dose delivery so that the friction brake provides a rotation brake between the first member and the second member. The dosing mechanism further comprises a hard stop that acts between the first member and the second member during dose delivery, wherein the hard stop is configured to restrict a movement of the first contact section towards the second contact section during dose delivery and to limit the pressure applied to the friction brake.

The hard stop thus prevents an uncontrolled compression of the friction brake and reliably defines the axial position of the first member with respect to the second member. This increases dosing accuracy with respect to embodiments that do not feature such a hard stop and in which the hardness of the friction brake solely defines the relative axial positions of the first and second member. Furthermore, variations of the hardness of the friction brake, for example due to production tolerances and/or aging, do not compromise dosing accuracy.

In addition, the hard stop allows to limit the pressure applied to the friction brake to values that ensure that the friction brake reliably disengages after having completed dose delivery. This prevents the friction brake from inhibiting or impeding a subsequent dose setting operation.

The hard stop may be configured separate from the first and/or second contact section. For example, the hard stop may be axially and/or radially displaced with respect to the first and/or second contact section. This allows separating engagement of the hard stop from engagement of the friction brake.

The hard stop may restrict movement of the first contact section towards the second contact section by stopping relative movement between the first contact section and the second contact section. This may result in the contact sections initially moving towards each other at the beginning of a dose delivery operation and to then stop on engagement of the hard stop. Alternatively, the contact sections may already be prevented from moving relative to each other at the beginning of the dose delivery operation.

The first contact section may be configured as one of a contact surface, a contact edge, and a contact corner of a first contact structure. Likewise, the second contact section may be configured as one of a contact surface, a contact edge, and a contact corner of a second contact structure. The first contact section may be integrally formed with the first member and/or the second contact section may be integrally formed with the second member.

The dosing mechanism may comprise a piston rod that is configured to move into a medicament holder attachable to the dosing mechanism to deliver the dose from the medicament holder. The axial force then may be transferred from the first member to the piston rod via the second member. The dosing mechanism may be configured to non-releasably or releasably attach to the medicament holder.

The medicament holder may be configured as a container holder that receives a container, such as a cartridge, containing the medicament in its interior. Alternatively, the medicament holder may comprise a compartment holding the medicament.

The piston rod may be rotationally fixed with respect to one of the first member and the second member at least during dose delivery. For example, the piston rod may be permanently rotationally fixed with respect to the one of the first member and the second member. The one of the first member and the second member may, for example, be the first member.

The other one of the first member and the second member, such as the second member, may be configured to rotate with respect to the piston rod during dose setting and may be configured to remain rotationally stationary with respect to the piston rod during dose delivery. The other one of the first member and the second member may be coupled to the piston rod via a threaded connection. For example, the other one of the first member and the second member may engage with the piston rod via the threaded connection. With alternative embodiments, the threaded connection may also be provided at one or more intermediate threaded parts that act between the piston rod and the other one of the first member and the second member.

The first member and the second member may be configured to move axially with respect to the piston rod during dose setting and to remain axially stationary with respect to the piston rod during movement of the piston rod for delivering the set dose. The piston rod thereby may move with respect to a housing of the dosing mechanism.

The dosing mechanism may comprise a housing and one of the first member and the second member, such as the first member, may be rotationally fixed with respect to the housing at least during dose setting. For example, the one of the first member and the second member may be permanently rotationally fixed with respect to the housing.

The dosing mechanism may comprise a dose setting element that is configured to be gripped and actuated by a user to set the dose. During dose setting, one of the first member and the second member, such as the second member, may be rotationally fixed to the dose setting element.

The dosing mechanism may comprise a first threaded connection that defines an axial position of the first member with respect to the housing and a second threaded connection that defines an axial position of the second member with respect to the piston rod. The first threaded connection may act between a first threaded part and the housing and the second threaded connection may act between a second threaded part and the piston rod.

The first threaded part and the second threaded part may be rotationally fixed with respect to each other during dose setting. Furthermore, the first threaded part and the second threaded part may be rotationally movable with respect to each other during dose delivery. The dosing mechanism may comprise a clutch that is configured to rotationally fix the first and second threaded part with each other during dose setting and to rotationally decouple the first threaded part from the second threaded part during dose delivery. During dose setting, the dose setting element may be rotationally fixed with respect to both the first threaded part and the second threaded part.

With some embodiments, the first threaded part may be configured as a dose member that is coupled between the first member and the housing. The dose member may be axially movable or axially fixed with respect to the first member. With other embodiments, the first threaded part may be formed by the first member. The first member then may be threadedly engaged with an intermediate member, such as a dose member, that acts between the first member and the housing. The intermediate member may be axially fixed and rotationally movable with respect to the housing.

With some embodiments, the second threaded part may be formed by the second member. Alternatively, the second threaded part may be formed by an intermediate member that is rotationally fixed to the second member. The intermediate member then may either be axially movable or axially fixed with respect to the second member.

The dosing mechanism may comprise a dose definition mechanism that is configured to define a dose to be delivered with the dosing mechanism. The dose definition mechanism thereby may define a rotational position of the second member with respect to the first member that corresponds to the dose. In general, the dose definition mechanism may be configured to define at least one dose. It thus may only define a single dose. Alternatively, it may be configured to define multiple doses.

The second member and/or the dose setting element may be configured to rotate with respect to the first member by at least one full revolution during dose setting. Alternatively, rotation of the second member and/or the dose setting element may be restricted to less than a full revolution during dose setting.

According to an embodiment, the axial force is transferred from the first member to the second member via the hard stop while the hard stop is restricting the movement. The force exerted on the second member then does not depend on the hardness or elasticity of the friction member once the hard stop has limited relative movement between the first and second member.

According to an embodiment, the hard stop has a first stop part axially fixed to the first member and a second stop part axially fixed to the second member, wherein engagement of the first stop part with the second stop part during dose delivery restricts the movement of the first contact section with respect to the second contact section. The first stop part thereby may be configured separate from the first contact section and/or the second stop part may be configured separate from the second contact section. The first stop part may be configured as one of a surface, an edge, and a corner of a first stop element. Likewise, the second stop part may be configured as one of a surface, an edge, and a corner of a second stop element.

According to an embodiment, the first stop part is integrally formed with the first member and/or the second stop part is integrally formed with the second member. The first stop part then may be formed by the first member and the second stop part may be formed by the second member.

According to an embodiment, at least one of the first stop part and the second stop part circumferentially surrounds the longitudinal axis. For example, the at least one of the first stop part and the second stop part may form a ring-like structure surrounding the longitudinal axis.

According to an embodiment, the hard stop is configured as an axial stop. This provides a rigid construction of the hard stop that is adapted to reliably transfer axial forces.

According to an embodiment, the first stop part is configured to move against the second stop part parallel to the longitudinal axis upon engagement of the hard stop. Such a construction provides a simple configuration of an axial stop.

According to an embodiment, the hard stop has a first stop surface axially fixed to the first member and a second stop surface axially fixed to the second member, wherein the first stop surface and the second stop surface are orientated perpendicular to the longitudinal axis. The first stop surface then may form the first stop part and the second stop surface may form the second stop part.

According to an embodiment, the hard stop defines a maximal compression of the friction brake. This limits axial movement of the first member with respect to the second member upon engagement of the friction brake and allows for high dose accuracy.

According to an embodiment, the maximal compression is at most 0.2 mm, such as at most 0.15 mm, at most 0.1 mm, at most 0.09 mm, at most 0.08 mm or at most 0.07 mm. The maximal compression may be at most 20%, such as at most 15%, at most 10%, at most 9%, at most 8% or at most 7% of a height of the friction brake, such as a height of the friction brake along a longitudinal axis of the dosing mechanism.

According to an embodiment, the first contact section circumferentially extends around the longitudinal axis and/or the second contact section circumferentially extends around the longitudinal axis. This equally and symmetrically distributes a pressure applied by the axial force to the friction brake.

According to an embodiment, the first contact section is configured as a surface that is perpendicular to the longitudinal axis and/or the second contact section is configured as a surface that is perpendicular to the longitudinal axis. Such a configuration facilitates disengagement of the first and second contact section upon axially moving the first member away from the second member.

According to an embodiment, the first contact section is configured as a first surface and the second contact section is configured as a second surface, wherein the first surface is parallel to the second surface.

According to an embodiment, the friction brake is configured as a separate friction member that is placed in between the first contact section and the second contact section. This allows the friction brake to have a different material than the first contact section and/or the second contact section. Furthermore, it allows the friction brake to have a different shape than the first contact section and/or the second contact section. As a consequence, elastic and/or frictional parameters of the friction brake may be adapted over a wide range.

According to an embodiment, the friction brake is configured as a ring-like structure, such as a torus or a washer. For example, the friction brake may be configured as an O-ring. Such parts are easily available as ready-made parts and allow for simple and cost-effective manufacture of the dosing mechanism.

According to an embodiment, the hard stop defines a gap between the first contact section and the second contact section during dose delivery, wherein the gap accommodates the friction brake. A width of the gap then may define the compression of the friction brake.

According to an embodiment, the gap is an axial gap. The gap then may separate the first and second contact section along the longitudinal axis.

According to an embodiment, the first member does not axially overlap with the friction brake in between the friction brake and the second contact section of the second member. Additionally or alternatively, the second member may not axially overlap with the friction brake in between the friction brake and the first contact section of the first member. Consequently, the entire cross section of the friction brake perpendicular to the longitudinal axis may be exposed to the first contact section and/or the second contact section. Furthermore, a lack of an axially overlap may reduce the probability that the first contact section interferes with the second member upon the first contact section engaging with the friction brake. Likewise, a probability that the second contact section interferes with the first member upon the second contact section engaging with the friction brake also may be reduced.

According to an embodiment, one of the first member and the second member forms a support member, wherein the friction brake is located on an outer surface of the support member. For example, the second member may form the support member.

The support member may have a cylindrical portion or body and the friction brake may be located at the cylindrical portion or body. For example, the friction brake may comprise an opening to receive the cylindrical portion or body.

According to an embodiment, the friction brake is axially retained on the support member, for example by a friction fit. The friction brake may comprise an elastic material and the friction brake may be mounted to the support member in a stretched state. This further retains the friction brake on the support member.

The first contact section may be provided at a cylindrical portion or body of the first member. For example, the first contact section may be provided at a cylindrical surface, such as an outer or inner surface, of the first member. For example, the first contact section may be provided at a contact structure that protrudes from the cylindrical surface.

Likewise, he second contact section may be provided at a cylindrical portion or body of the second member. For example, the second contact section may be provided at a cylindrical surface, such as an outer or inner surface, of the second member. For example, the second contact section may be provided at a contact structure that protrudes from the cylindrical surface.

According to an embodiment, one of the first member and the second member forms an inner member and the other one of the first member and the second member forms an outer member, wherein the outer member at least partly surrounds the inner member. For example, the outer member may surround the contact section of the inner member. The outer member may be formed by the first member and the inner member may be formed by the second member.

According to an embodiment, the outer member radially overlaps with the friction brake, wherein, for example, the friction brake is located in an end cavity of the outer member. The end cavity may be located at an axial end of the outer member. For example, the end cavity may be located at a proximal end of the outer member. The end cavity may be open at the axial end. For example, the end cavity may form a cylindrical cavity at an end of the outer member.

The friction brake may be mounted to the inner member. The friction brake then may contact a surface of the inner member, such as an outer surface of the inner member.

According to an embodiment, the outer member is radially spaced from the friction brake by a clearance. This prevents the outer member from touching and moving the friction brake when shifting with respect to the inner member, for example along the longitudinal axis.

According to an embodiment, the clearance is smaller than the radial height of the friction brake. This may prevent the friction brake from snapping over a contact structure that axially retains the friction brake on the inner member by limiting radial movement of the friction brake at the outer member.

According to an embodiment, the clearance is at most 0.5 times, such as at most 0.4 times, or at most 0.3 times a radial height of the friction brake.

According to an embodiment, the inner member forms the support member. The friction brake then is mounted on the inner member and surrounded by the outer member.

According to an embodiment, the first contact section axially overlaps with the friction brake over a first radial height that is at least 0.5 times, such as at least 0.6 times, at least 0.7 times or at least 0.8 times a radial height of the friction brake. Additionally or alternatively, the second contact section may axially overlap with the friction brake over a second radial height that is at least 0.5 times, such as at least 0.6 times, at least 0.7 times or at least 0.8 times a radial height of the friction brake. With a ring-like friction brake, the radial height of the friction brake may be the radial thickness of the ring, that is the difference between an outer diameter and an inner diameter of the friction brake.

According to an embodiment, the first contact structure comprising the first contact section axially overlaps with the friction brake over a first radial height that is at least 0.5 times, such as at least 0.6 times, at least 0.7 times or at least 0.8 times a radial height of the friction brake. Additionally or alternatively, the second contact structure comprising the second contact section may axially overlap with the friction brake over a second radial height that is at least 0.5 times, such as at least 0.6 times, at least 0.7 times or at least 0.8 times a radial height of the friction brake.

A large overlap of the individual contact sections and/or contact structures with the friction brake in the radial direction helps to prevent the friction brake from axially shifting along the longitudinal axis.

According to an embodiment, the friction brake is located in a cavity formed between the first member and the second member, wherein the cavity is radially bounded by the first member and the second member and axially bounded by a first contact structure comprising the first contact section and a second contact structure comprising the second contact section. Furthermore, a first radial spacing between the first contact structure and the second member is smaller than the radial height of the friction brake and/or a second radial spacing between the second contact structure and the first member is smaller than the radial height of the friction brake. This securely fixes the friction brake in between the first and second member.

According to an embodiment, the friction brake is configured as an elastic member, wherein, for example, the friction brake comprises rubber or consists of rubber. The rubber may be a natural rubber or it may be a synthetic rubber. For example, the rubber may be nitrile butadiene rubber (NBR).

According to an embodiment, a hardness of the friction brake is lower than a hardness of the first contact section and/or a hardness of the second contact section. This provides a well-defined compression of the friction brake in between the first and second contact sections that is primarily given by the hardness of the friction brake.

According to an embodiment, the friction brake has at most a Shore A hardness of 90, such as at most a Shore A hardness of 85, at most a Shore A hardness of 80, or at most a Shore A hardness of 75. These values have proven to be suited for typical dosing mechanisms.

According to an embodiment, the dosing mechanism comprises a further stop, wherein the further stop acts between the first member and the second member and wherein the further stop restrains axial movement of the first contact section away from the second contact section during dose setting. The further stop may be configured as an axial stop. It may comprise a first further stop part and a further second stop part that engage with each other to restrict axial movement of the first contact section away from the second contact section. The first further stop part may be integrally formed with the first member and/or the second stop part may be integrally formed with the second member.

According to an embodiment, one of the first member and the second member, such as the first member, is permanently rotationally fixed to the housing of the dosing mechanism. The one of the first member and the second member may, for example, be axially movable with respect to the housing. It may, for example, be connected to the housing via a splined connection.

According to an embodiment, the dosing mechanism comprises a latching mechanism that defines discrete rotational positions of the second member with respect to the first member. The first member thereby is permanently rotationally fixed to a first latching part of the latching mechanism and the second member is permanently rotationally fixed to a second latching part of the latching mechanism. Furthermore, the first latching part and the second latching part releasably engage with each other upon relative rotation of the first member relative to the second member during dose setting and the latching mechanism provides an additional rotation brake preventing relative rotation of the first member and the second member during dose delivery.

The latching mechanism may provide the dose definition mechanism that defines at least one rotational position of the first member with respect to the second member that corresponds to at least one settable dose.

According to an embodiment, the second latching part is axially fixed to the second member and the second contact section and the second latching part are separate from each other. This prevents the first contact section from interfering with the second latching part via the friction brake.

For example, the second contact section may be axially displaced with respect to the second latching part. A gap may be formed between the second latching part and the second contact section. Both the second contact section and the second latching part may protrude radially from the second member.

In general, the dosing mechanism may comprise a dose definition mechanism that allows a user of the device to set at least one dose of medicament for delivery. For example, the dose definition mechanism may be configured to allow only a single predetermined dose to be set. Alternatively, the dose definition mechanism may also be configured to allow a multitude of differing doses to be set by the user, such as two or more differing doses. The dose definition mechanism may allow to set multiple doses that differ by equidistant dose steps.

The dosing mechanism may be configured as a single-use mechanism that allows to set a dose only once and subsequently prevents a user from setting and delivering further doses. The dosing mechanism may also be configured as a multi-use mechanism that allows to repeatedly set doses for delivery.

The dosing mechanism may be configured as a disposable mechanism that is disposed of after having delivered a last dose from a medicament container attached to the dosing mechanism. With other embodiments, the dosing mechanism may be configured as a reusable mechanism that is configured to detach from the medicament holder after having delivered a last dose from the medicament holder and to attach to a medicament holder comprising at least one additional dose to be delivered by the dosing mechanism.

With the dosing mechanism, a delivery of the medicament may require an axial force to be exerted by a user, for example on an actuation member of the dosing mechanism. For example, the user may have to exert the axial force with respect to the housing. With other embodiments, the dosing mechanism may be configured as an automatic mechanism that is configured to deliver the dose of medicament without requiring a transfer of a force provided by the user to a member that is in contact with the medicament and moves for delivering the medicament, such as to a piston sealing a compartment comprising the medicament.

In a further aspect, the present disclosure is directed at a dosing mechanism for a medicament delivery device, the dosing mechanism comprising a first member, a second member and a friction brake. The friction brake thereby is located in between a first contact section of the first member and a second contact section of the second member and the first member is configured to rotate with respect to the second member around a longitudinal axis during setting of a dose of medicament to be delivered. Furthermore, the first member is configured to transfer an axial force to the second member in a proximal direction parallel to the longitudinal axis during dose delivery to deliver the dose. The first contact section is configured to press the friction brake towards the second contact section during dose delivery so that the friction brake provides a rotation brake between the first member and the second member.

The dosing mechanism then may not comprise the hard stop.

In another aspect, the present disclosure is directed at a medicament delivery device having one of the dosing mechanisms according to the present disclosure.

The medicament delivery device may be configured as an injection device, such as a pen injection device. A medicament holder containing the medicament to be delivered may be configured to receive a cannula at its proximal end to deliver the medicament through the cannula.

The present disclosure also is directed at a medicament delivery device having
a medicament holder, a housing, and a cap, wherein the medicament holder is configured to releasably attach to the housing via a connector by rotating the medicament holder around a longitudinal axis with respect to the housing and wherein the cap is configured to releasably attach to the medicament holder to at least partly cover the medicament holder.

The medicament delivery device may have one of the dosing mechanisms according to the present disclosure. Alternatively, the medicament delivery device may also have dosing mechanisms that are different from the dosing mechanisms according to the present disclosure.

According to an embodiment, the medicament holder comprises a first blocking element of a blocking mechanism that is configured to engage with a second blocking element of the blocking mechanism, the second blocking element being provided at the housing. The blocking mechanism thereby is interlocked by the cap to rotationally lock the medicament holder to the housing when the cap is attached to the medicament holder and the blocking mechanism is unlocked to allow rotation of the medicament holder with respect to the housing when the cap is detached from the medicament holder.

Such first and second blocking elements prevent rotation of the medicament holder upon detachment of the cap from the medicament holder.

According to an embodiment, one of the first blocking element and the second blocking element is attached to a flexible element that is configured to bend away from the other one of the first blocking element and the second blocking element to allow the rotation of the medicament holder with respect to the housing. Furthermore, attachment of the cap to the medicament holder blocks the flexible element from bending. Exemplarily, the first blocking element may be attached to the flexible element.

According to an embodiment, the cap is configured to engage with the flexible element to block the flexible element from bending.

According to an embodiment, the flexible element is configured to bend radially outwards to allow the rotation of the medicament holder with respect to the housing. The medicament holder then may be configured to move the flexible element into the cap to interlock the blocking mechanism.

According to an embodiment, the flexible element is provided at the medicament holder.

According to an embodiment, the flexible element is integrally formed with the medicament holder. For example, the flexible element may be formed within an outer wall of the medicament holder. The flexible element may be configured as a free-standing structure that is surrounded by a gap formed in the medicament holder, such as in the outer wall of the medicament holder.

According to an embodiment, the flexible element is accessible at an outer surface of the medicament holder. The outer surface may form an outer surface of the outer wall of the medicament holder.

According to an embodiment, the flexible element has a fixed first end and a free second end, wherein the one of the first and second blocking element is attached to the second end and wherein the first end is located opposite the second end along the longitudinal axis. Compared to a flexible element that is orientated circumferentially with the fixed end and the free and being located at the same axial position of the medicament holder, the flexible element being orientated axially provides equal resistance to the one of the first and second blocking element irrespective of a rotation direction of the first blocking element with respect to the second blocking element.

According to an embodiment, the blocking mechanism is configured as a radial block,
wherein the first blocking element is configured to engage with the second blocking element by rotating with respect to the second blocking element around the longitudinal axis.

According to an embodiment, one of the first blocking element and the second blocking element is configured as a radial protrusion and the other one of the first blocking element and the second blocking element is configured as a radial detent that is configured to receive the radial protrusion. For example, the first blocking element of the medicament holder may be configured as the radial protrusion and the second blocking element of the housing may be configured as the radial detent.

According to an embodiment, the radial detent has a larger length in a circumferential direction than the radial protrusion. This allows the blocking mechanism to rotationally lock the medicament holder to the housing over a range of relative rotational positions of the medicament holder with respect to the housing. The range thereby may include all rotational positions that the medicament holder takes up during the lifetime of the medicament delivery device. Those rotational positions may, for example, be given by a maximum wear of the connector for attaching the medicament holder to the housing.

According to an embodiment, the radial detent has a first side surface in a first circumferential direction and a second side surface in a second circumferential direction opposite the first circumferential direction, wherein the first side surface is engaged by the radial protrusion upon rotating the medicament holder to detach the medicament holder from the housing. The first side surface thereby is steeper than the second side surface. this allows for reliable interlocking of the blocking mechanism and at the same time provides space to account for the rotational positions that the medicament holder may take up during the lifetime of the medicament delivery device, for example due to wear of the connector to the housing.

The first blocking element may be located in a protruding axial section, in which an outer surface of the medicament holder has a larger radial extent than in an adjacent recessed axial section. The first blocking element then may be configured to disengage from the inner surface of the cap only when the inner surface of the cap leaves the protruding axial section. This prevents the inner surface of the cap from interfering with the medicament holder after having unlocked the blocking mechanism. For example, the recessed section may extend from the protruding axial section up to a proximal end of the medicament holder.

According to an embodiment, the connector comprises a first connection element provided at the medicament holder, wherein the first connection element is configured as a female connector that is configured to receive a second connection element provided at the housing. The second connection element than may be configured as a male connector. For example, the first connection element may be configured as an inner thread and the second connection element may be configured as an outer thread.

According to an embodiment, the connector is configured as a threaded connector. With alternative embodiments, the connector may, for example, be configured as a bayonet lock.

According to an embodiment, the medicament holder comprises a first cap lock element that is configured to engage with a second cap lock element of the cap to axially hold the cap at the medicament holder. The first cap lock element and the second cap lock element may be configured as a snap fit connection.

According to an embodiment, a cap lock formed by the first cap lock element and the second cap lock element is configured as an axial lock that does not restrict rotation of the cap with respect to the medicament holder.

According to an embodiment, the medicament holder comprises a first aligning part of a cap alignment mechanism and the cap comprises a second aligning part of the cap alignment mechanism, wherein the first aligning part is configured to engage with the second aligning part upon attachment of the cap to the medicament holder to attach the cap in a predefined rotational orientation to the housing.

According to an embodiment, the first aligning part and/or the second aligning part comprise a lead-in taper that narrows in a distal direction parallel to a longitudinal axis.

According to an embodiment, both the first aligning part and the second aligning part comprise the lead-in taper, wherein a first taper angle of the first aligning part is larger than a second taper angle of the second aligning part.

According to an embodiment, the lead-in taper is configured as a non-self-locking taper,
wherein the lead-in taper forces the cap to move in a proximal direction parallel to the longitudinal axis upon rotating the cap while being fully attached to the housing. This allows a user to detach the cap by rotating it with respect to the housing and thus provides a rotate-to-unlock mechanism.

According to an embodiment, one of the first aligning part and the second aligning part is configured as a radial protrusion and the other one of the first aligning part and the second aligning part is configured as a radial depression that receives the radial protrusion upon attachment of the cap to the housing.

According to an embodiment, the radial depression has an open proximal end. The open proximal end may be configured to receive the radial protrusion upon axially moving the cap onto the medicament holder.

According to an embodiment, the radial depression comprises a first radial sidewall and the radial protrusion comprises a second radial sidewall, wherein the first radial sidewall and/or the second radial sidewall are angled with respect to a radial plane perpendicular to the longitudinal axis and wherein the first radial sidewall is configured to slide along the second radial sidewall upon attachment of the cap to the housing to align the cap in the predefined rotational orientation.

According to an embodiment, the first radial sidewall and/or the second radial sidewall are angled with respect to the longitudinal axis. This may provide the lead-in taper.

According to an embodiment, the first radial sidewall and the second radial sidewall comprise an undercut in a cross-sectional plane perpendicular to the longitudinal axis. Such undercuts prevent disengagement of first and second aligning parts.

According to an embodiment, the cap comprises a first member comprising the second aligning part and a second member non-releasably joined to the first member, for example by an adhesive bond. The first member thereby may be glued to the second member. The first member may be configured as a cap end element and the second member may be configured as a cap body.

According to an embodiment, the first member comprises a further first aligning part of a body alignment mechanism and the second member comprises a further second aligning part of the body alignment mechanism, wherein the engagement of the further first aligning part with the further second aligning part is configured to rotationally align the first member with respect to the second member. Exemplarily, the further first aligning part may be configured as a radial protrusion and the further second aligning part may be configured as a radial recess, such as a radial notch, that receives the radial protrusion.

According to an embodiment, the first member forms an inner part and the second member forms an outer part at least partly surrounding the inner part.

According to an embodiment, the first member comprises a plastic material, for example consists of the plastic material, wherein the second member comprises a metal material, for example consists of the metal material.

According to an embodiment, the cap comprises a cap body and a cap end element,
wherein the cap end element is located at a proximal end of the cap body and wherein the second aligning part is formed at the cap end element.

According to an embodiment, the first aligning part is made from a plastic material, such as a thermoplastic material. Additionally or alternatively, the second aligning part may be made from a plastic material, such as a thermoplastic material. This provides for cost efficient and easy manufacture for the first and/or second aligning part.

According to an embodiment, the first aligning part is configured as a molded part, such as an injection molded part. Additionally or alternatively, the second aligning part may be configured as a molded part, such as an injection molded part.

The first aligning part and the second aligning port may be configured to only engage when the cap has been moved over the medicament holder by at least 0.5 times, such as at least 0.6 times or at least 0.7 times the total length of the medicament holder covered by the cap in a fully attached state.

According to an embodiment, the first aligning part is located at a proximal end of the medicament holder and/or the second aligning part is located at a proximal end of the cap. The first aligning part may be located in a proximal half, such as a proximal quarter of the medicament holder and/or the second aligning part may be located in a proximal half, such as a proximal quarter of the cap.

The present disclosure is also directed at a medicament holder having the features of the medicament holder of the medicament delivery device according to the present disclosure. Furthermore, the present disclosure relates to a housing having the features of the housing of the medicament delivery device according to the present disclosure.

### DRAWINGS

Exemplary embodiments and functions of the present disclosure are described herein in conjunction with the following drawings, showing schematically:
- Fig. 1: a first side view of a medicament delivery device according to the present disclosure having a dosing mechanism according to the present disclosure;
- Fig. 2: a second side view of the medicament delivery device;
- Fig. 3: a perspective view of the medicament delivery device with a cap of the medicament delivery device removed;
- Fig. 4: a first side view of the medicament delivery device with the cap removed;
- Fig. 5: a second side view of the medicament delivery device with the cap removed;
- Fig. 6: a first cross-sectional side view of a medicament holder of the medicament delivery device with the cap attached to the medicament holder;
- Fig. 7: a second cross-sectional side view of the medicament holder with the cap attached to the medicament holder;
- Fig. 8: a side view of a cap end element of the cap;
- Fig. 9: a perspective view of the cap end element;
- Fig. 10: a perspective view of a cap body of the cap;
- Fig. 11: a perspective cross-sectional view of the cap body;
- Fig. 12: a perspective view of the medicament holder;
- Fig. 13: a side view of the medicament holder;
- Fig. 14: a perspective cross-sectional view of the medicament holder;
- Fig. 15: a cross-sectional view of the cap and the medicament holder in a plane perpendicular to a longitudinal axis;
- Fig. 16: a perspective view of an outer housing of the dosing mechanism;
- Fig. 17: a side view of the outer housing of the dosing mechanism;
- Fig. 18: a perspective cross-sectional view of the outer housing;
- Fig. 19: a top view of the outer housing from a proximal end;
- Fig. 20: a detailed cross-sectional side view of the medicament holder attached to the dosing mechanism and the cap attached to the medicament holder;
- Fig. 21: a cross-sectional top view of the medicament holder attached to the dosing mechanism and the cap attached to the medicament holder;
- Fig. 22: an exploded view of the medicament delivery device and the dosing mechanism;
- Fig. 23: a cross-sectional side view of the dosing mechanism in a dose setting state with the dose set;
- Fig. 24: a cross-sectional side view of the dosing mechanism in the dose setting state with a maximum dose set;
- Fig. 25: a cross-sectional side view of the dosing mechanism in a dose delivery state with the maximum dose set;
- Fig. 26: a perspective view of an inner housing of the dosing mechanism;
- Fig. 27: a cross-sectional perspective view of the inner housing of the dosing mechanism with a cut plane parallel to a longitudinal axis;
- Fig. 28: a top view of the inner housing in a distal direction;
- Fig. 29: perspective view of a piston rod guide of the dosing mechanism in a proximal direction;
- Fig. 30: a perspective view of the piston rod guide in a distal direction;
- Fig. 31: a side view of the piston rod guide;
- Fig. 32: a top view on a proximal end of the piston rod guide;
- Fig. 33: a cross-sectional side view of a first member, a second member and a friction brake of the dosing mechanism;
- Fig. 34: a cross-sectional side view of the first member, the second member and the friction brake in a cut plane perpendicular to a cut plane of Fig. 33;
- Fig. 35: a perspective view of a nut of the dosing mechanism in a distal direction;
- Fig. 36: a perspective view of the nut in a proximal direction;
- Fig. 37: a perspective cross-sectional view of the nut in a cross-sectional plane parallel to a longitudinal axis;
- Fig. 38: a top view of a proximal end of the nut;
- Fig. 39: a top view of a distal end of the nut;
- Fig. 40: a perspective view of a driver of the dosing mechanism in a distal direction;
- Fig. 41: a perspective view of the driver in a proximal direction;
- Fig. 42: a cross-sectional perspective view of the driver;
- Fig. 43: a cross-sectional view of the driver, the nut, the inner housing, a dose member, and a piston rod of the dosing mechanism in a cut plane perpendicular to the longitudinal axis;
- Fig. 44: a perspective view of a connector of the dosing mechanism in a proximal direction
- Fig. 45: a perspective cross-sectional view of the connector in the proximal direction in a cut plane parallel to a longitudinal axis;
- Fig. 46: a top view of a distal end of the connector;
- Fig. 47: a perspective view of a dose member of the dosing mechanism;
- Fig. 48: a side view of the dose member of the dosing mechanism;
- Fig. 49: a perspective cross-sectional view of the dose member in a cut plane parallel to a longitudinal axis;
- Fig. 50: a perspective view of a dose setting element of the dosing mechanism in a distal direction;
- Fig. 51: a perspective view of the dose setting element in a proximal direction;
- Fig. 52: a perspective cross-sectional view of the dose setting element in a cut plane parallel to a longitudinal axis;
- Fig. 53: a perspective view of a button ring of the dosing mechanism in a distal direction;
- Fig. 54: a perspective cross-sectional view of the button ring in a cut plane parallel to a longitudinal axis;
- Fig. 55: a perspective view of a button disc of the dosing mechanism in a distal direction;
- Fig. 56: a perspective cross-sectional view of the button disc in a cut plane parallel to a longitudinal axis;

### DETAILED DESCRIPTION

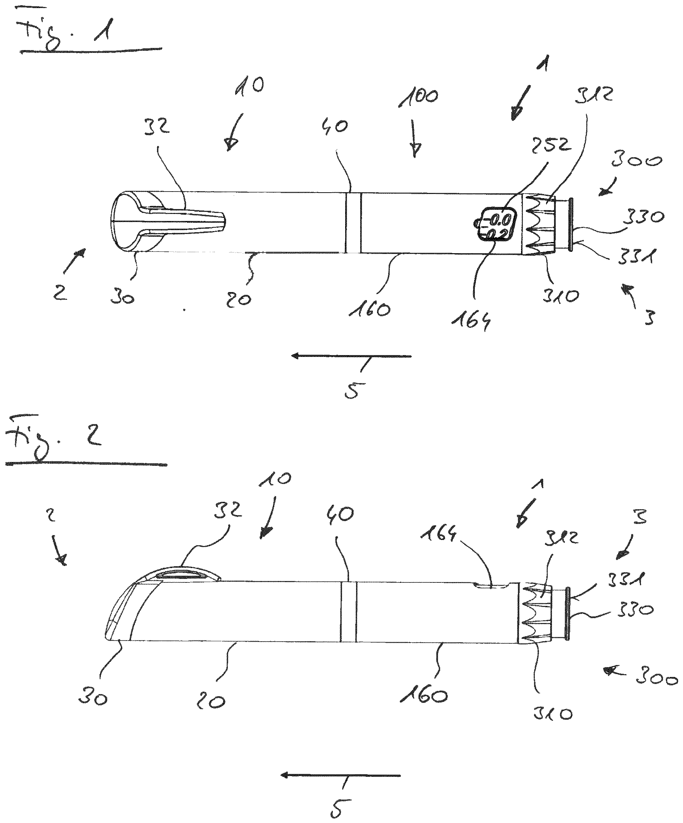

**Figs. 1** and **2** depict side views of a medicament delivery device 1 according to the present disclosure. The medicament delivery device 1 is configured as an injection device, namely as a pen injection device. The injection device 1 extends from a distal end 3 in a proximal direction 5 to a proximal end 2.

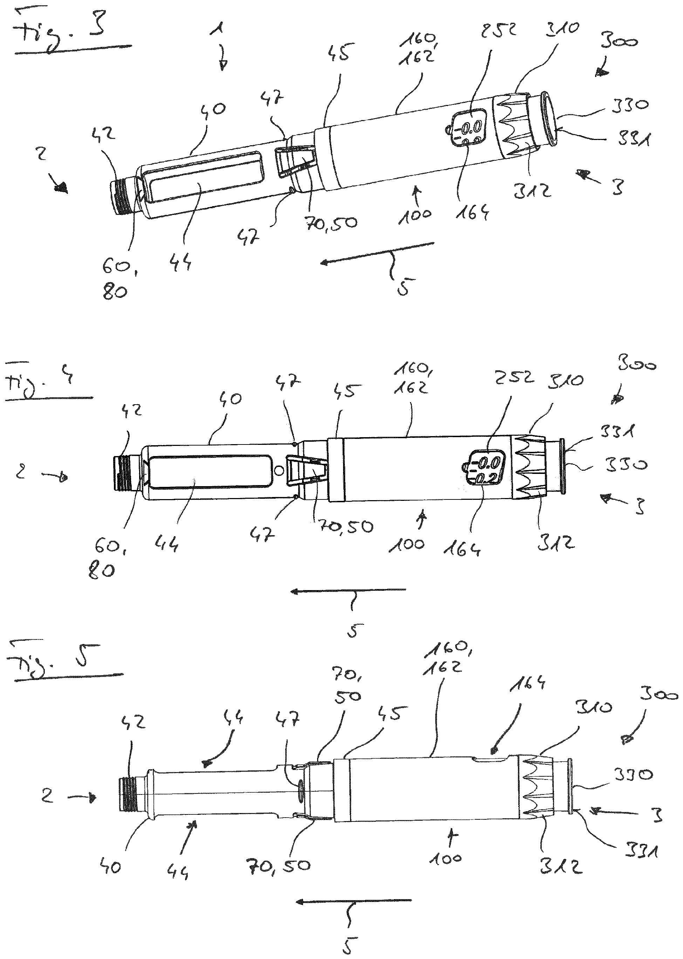

While Figs. 1 and 2 show the medicament delivery device 1 with a cap 10 attached to a dosing mechanism 100 of the medicament delivery device 1, **Figs. 3** to **5** depict the medicament delivery device 1 with the cap 10 removed from the dosing mechanism 100.

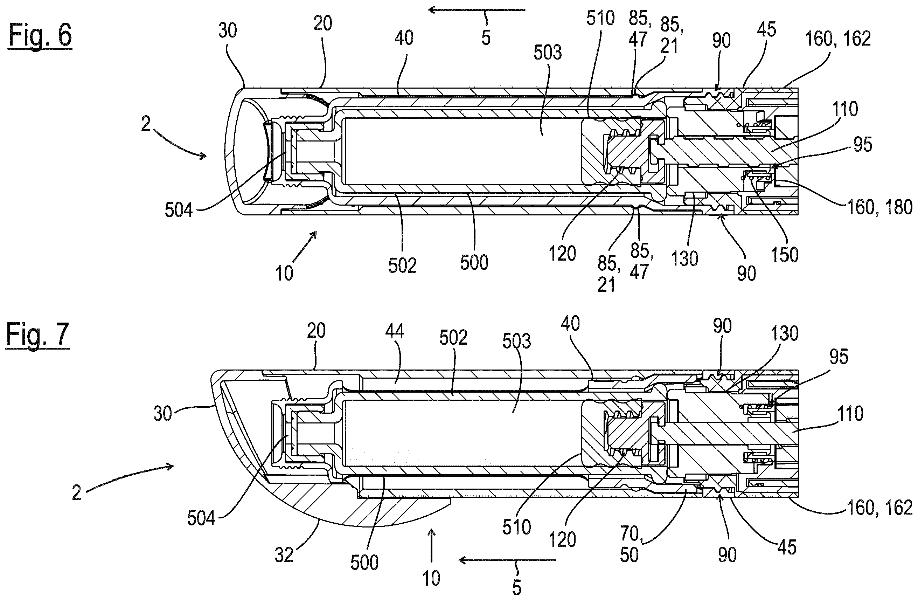

**Figs. 6** and **7** show cross-sectional side views of the medicament holder 40 attached to a housing 160 of the dosing mechanism 100 and the cap 20 attached to the medicament holder 40.

The medicament holder 40 is releasably attached to a proximal end of the dosing mechanism 100. It connects to the housing 160, namely to an outer housing 162 of the housing 160, via a connector 90. The connector 90 is configured as a threaded connector.

The medicament holder 40 is configured to receive a medicament container 500, such as a cartridge, within an interior cavity and to attach the medicament container 500 to the housing 160. The medicament container 500 comprises a container body 502 that surrounds a medicament compartment 503 containing the medicament. A distal end of the medicament compartment 503 is sealed by a movable piston 510 and a proximal end of the medicament compartment 503 is sealed by a septum 504. The piston 510 and the septum 504 are made from an elastic material, such as a rubber. With alternative embodiments, the medicament compartment 503 may also be integrally formed with the medicament holder 40. The medicament holder 40 comprises two radially opposing windows 44 that allow for inspection of the medicament within the medicament holder 40.

At the proximal end 2, the medicament holder 40 comprises a needle connector 42 that is configured to secure a cannula to the medicament holder 40. Upon attachment of the cannula to the medicament holder 40, a fluid connection between the medicament container 500 and the cannula is established by piercing the septum 504 provided at a proximal end of the medicament container 500. The needle connector 42 is exemplarily configured as a threaded connector. With other embodiments, the needle connector 42 may also be configured as a bayonet lock, a Luer lock or the like.

With the drug delivery device 1, the cap 10 is connected to the housing 160 of the dosing mechanism 100 via the medicament holder 40. The cap 10 thereby only engages with the medicament holder 40 but not with the housing 160 or any other part of the dosing mechanism 100. A cap lock 85 acts between the cap 10 and the medicament holder 40. The cap lock 85 is configured to releasably hold the cap 10 at the medicament holder 40 in an axial direction.

On its outer surface, the cap 10 comprises a protruding part 32. The protruding part 32 extends parallel to the longitudinal axis 7 and generally protrudes in the radial direction. The protruding part 32 is configured to prevent rotation of the cap 10 and the dosing mechanism 100 attached to the cap 10 when being placed on a flat surface. Exemplarily, the protruding part 32 is configured as a longitudinal clip.

The cap 10 comprises a cap body 20 and a cap end element 30 that is attached to a proximal end of the cap body 20. The cap end element 30 forms a first member of the cap 10 and the cap body 20 forms a second member of the cap 10. The cap end element 30 is permanently glued to the cap body 20.

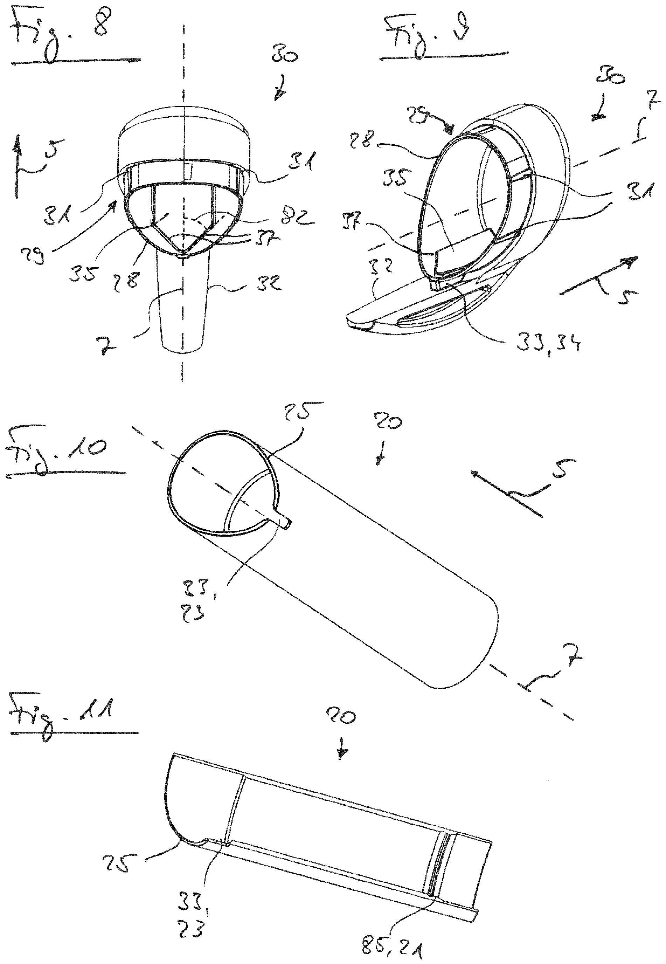

**Figs. 8** and **9** depict the cap end element 30 and **Figs. 10** and **11** depict the cap body 20.

The cap body 20 has a generally cylindrical shape and extends along a longitudinal axis 7. The cap body 20 is configured as a sleeve that is open at both longitudinal ends. The cap body 20 is configured as a machined part. It is made from metal. Exemplarily, the cap body 20 is configured as a lathed piece.

The cap end element 30 is made from a thermoplastic material. Exemplarily, it is configured as an injection molded part. The cap end element 30 closes the cap body 20 at its proximal end and forms an end cap of the cap body 20.

The cap end element 30 has a radially recessed section 29 that is received in an interior of the cap body 20. The cap end element 30 with the recessed section 29 forms an inner part of the cap 10 and the cap body 20 forms an outer part of the cap 10 that partly surrounds the inner part. The glue for attaching the cap end element 30 to the cap body 20 is provided between the recessed section 29 and the cap body 20. On its outer surface, the recessed section 29 comprises centering elements 31 that are configured as radially protruding ridges. The ridges are orientated parallel to the longitudinal axis 7. They taper radially down towards the longitudinal axis 7 in a distal direction opposite the proximal direction 5 to facilitate attachment of the cap end element 30 to the cap body 20.

The cap 10 comprises a body alignment mechanism 33 that defines a single predefined rotational orientation of the cap end element 30 with respect to the cap body 20 after attachment of the cap end element 30 to the cap body 20. The body alignment mechanism 33 comprises a first aligning part 34 provided at the cap end element 30 and a second aligning part 23 provided at the cap body 20. The second aligning part 23 is configured as a radial recess, namely as a radial notch, provided in an outer wall of the cap body 20 and the first aligning part 34 is configured as a radial protrusion provided at an outer wall of the cap end element 30. The second aligning part 23 is thereby accessible at an inside surface of the outer wall of the cap body 20 and the first aligning part 34 protrudes from an outer surface of the outer wall of the cap end element 30. Furthermore, the first aligning part 34 is provided at the recessed section 29 of the cap end element 30. Both the first aligning part 34 and the second aligning part 23 longitudinally extend along the longitudinal axis 7.

In addition to rotationally aligning the cap end element 30 with the cap body 20, the body alignment mechanism 33 also rotationally locks the cap end element 30 to the cap body 20. The body alignment mechanism 33 therefore also acts as a rotation lock between the cap body 20 and the cap end element 30.

In addition to the body alignment mechanism 33, the cap 10 comprises an additional body alignment mechanism that defines the rotational orientation of the cap body 20 with respect to the cap end element 30. A first alignment feature of the additional body alignment mechanism is provided by a distally facing surface 28 of the cap end element 30 and a second alignment feature of the additional body alignment mechanism is formed by a proximally facing surface 25 of the cap body 20. The first alignment feature is configured to engage with the second alignment feature when the cap end element 30 is attached to the cap body 20. Thereby, the first alignment feature and the second alignment feature are configured rotationally asymmetric with respect to the longitudinal axis 7.

Exemplarily, the distally facing surface 28 of the first alignment feature fully extends around the longitudinal axis 7 and the proximally facing surface 25 of the second alignment feature likewise fully extends around the longitudinal axis 7.

The distally facing surface 28 and the proximally facing surface 25 are configured to contact each other over their whole surface only in a single relative rotational orientation. The distally facing surface 28 thereby is exemplarily located around the circumference around the longitudinal axis 7 at varying axial positions along the longitudinal axis 7. Likewise, the proximally facing surface 25 is also located at varying axial positions along the longitudinal axis 7 around the circumference around the longitudinal axis 7.

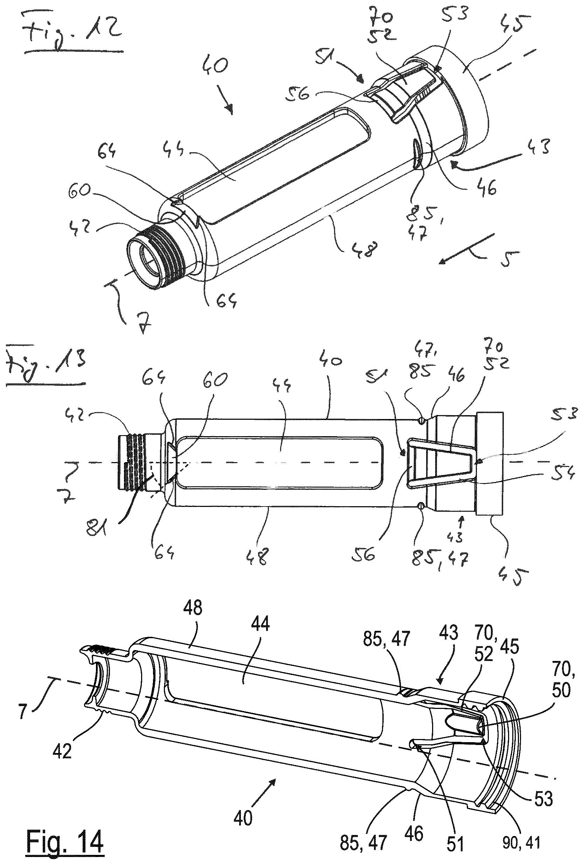

**Figs. 12** to **14** depict the medicament holder 40. The medicament holder 40 has a generally cylindrical outer body. After attachment, the cap 10 covers the entire outer body of the medicament holder 40 except for a distal portion of the medicament holder 40. The non-covered distal portion forms a cylindrical part. It comprises an outer surface 45 that is exposed in between the housing 160 of the dosing mechanism 100 and the cap 10 when the cap 10 is attached to the housing 160, see Figs. 1 and 2. As can be seen from Figs. 12 to 14, the cylindrical part forms a portion of the medicament holder 40 that has the largest extension in the radial direction.

A cap alignment mechanism 80 acts between the cap 10 and the housing 100. The cap alignment mechanism 80 is configured to define a single rotational orientation in which the cap 10 is attachable to the housing 160. With the medicament delivery device 1, the cap alignment mechanism acts between the cap 10 and the medicament holder 40.

The medicament holder 40 thereby comprises a first aligning part 60 of the cap alignment mechanism 80 that interacts with a second aligning part 35 of the cap alignment mechanism 80 to define the rotational orientation between the cap 10 and the housing 160.

The first aligning part 60 is located at the proximal end of the medicament holder 40 and distally from the needle connector 42. Furthermore, the first aligning part 60 is formed at a radial step of the medicament holder 40. The radial step connects a radially narrower section of the medicament holder 40 with a radially wider section, whereby the radially narrower section is located in the proximal direction from the radially wider section.

The first aligning part 60 is configured as a radial depression on the outer surface of the body of the medicament holder 40. The radial depression is open along the longitudinal axis 7 in both the proximal direction 5 and the distal direction. In the proximal direction 5, the radial depression terminates at the radial step and, in the distal direction, the radial depression terminates at one of the windows 44 provided in the outer body of the medicament holder 40.

The first aligning part 60 comprises a lead-in taper that narrows in the distal direction. The lead-in taper is exemplarily formed by first sidewalls 64 that delimit the first aligning part 60 in the circumferential direction around the longitudinal axis 7. The sidewalls 64 are tilted with respect to the longitudinal axis 7 and have a first taper angle 81 with respect to the longitudinal axis 7.

The second aligning part 35 of the cap alignment mechanism 80 is provided at the cap end element 30. It is located at an inner surface of the cap end element 30. Exemplarily, it is configured as a protrusion that protrudes from the inner surface. Furthermore, the second aligning part 35 is located at a proximal end of the cap 10.

The second aligning part 35 comprises a lead-in taper that narrows in the distal direction. Second sidewalls 37 of the second aligning part 35 are tilted with respect to the longitudinal axis 7. The second sidewalls 37 have a second taper angle 82 with the longitudinal axis 7. Both the first taper angle 81 and the second taper angle 82 are essentially constant along the longitudinal lengths of the first sidewalls 64 and the longitudinal lengths of the second sidewalls 37, respectively.

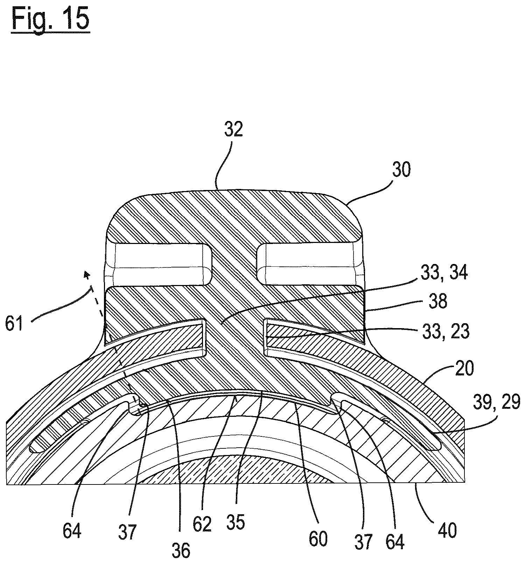

**Fig. 15** depicts a cross-sectional view of the cap 10 and the medicament holder 40 in a plane perpendicular to a longitudinal axis 7 through the cap alignment mechanism 80 and the body alignment mechanism 33.

With the cap alignment mechanism 80, a radially outwardly facing surface 62 of the first aligning part 60 faces a radially inwardly facing surface 36 of the second aligning part 35. The surfaces 62, 36 are generally aligned parallel to each other. The first sidewalls 64 of the first alignment feature have an undercut in the radial direction 61. Likewise, the first sidewalls 37 of the second alignment feature 35 comprise an undercut in the radial direction 61.

The recessed section 29 of the cap end element 30 forms an inner part 39 received within the cap body 20 and the protruding part 32 forms an outer part 38 that is positioned outside the cap body 20. The inner part 39 and the outer part 38 are connected by the first aligning part 34 of the body alignment mechanism 33. The first aligning part 34 is thereby located within the notch formed by the second aligning part 23 of the body alignment mechanism 33.

As can be seen from Figures 12 to 14, the cap lock 85 comprises a first cap lock element 47 at the medicament holder 40 that engages with a second cap lock element 21 provided at the cap 10, as shown in Fig. 11. The first cap lock element 47 is configured as a radial protrusion located on an outer surface of the medicament holder 40. The first cap lock element 47 is elongated in the circumferential direction and has a length along the circumferential direction that is larger than a width along the longitudinal axis 7. The length thereby is limited to less than a full revolution, namely to less than half a full revolution, such as less than a quarter of a full revolution.

The second cap lock element 21 is configured as a radial depression on an inside surface of the cap 10, namely on an inside surface of the cap body 20. The second cap lock element 21 is located along a circumferential direction and has a length in the circumferential direction that is larger than a width along the longitudinal axis 7. The second cap lock element 21 thereby extends over a full revolution around the longitudinal axis 7.

As can be seen from Fig. 6, the first cap lock element 47 engages with the second cap lock element 21 to axially retain the cap 10 on the medicament holder 40.

As can be seen from Fig. 14, the medicament holder 40 comprises a first connection element 41 of the connector 90 that connects the medicament holder 40 releasably to the housing 160, namely to the outer housing 162. The first connection element 41 thereby is located at the non-covered distal portion of the medicament holder 40 that is bounded by the outer surface 45.

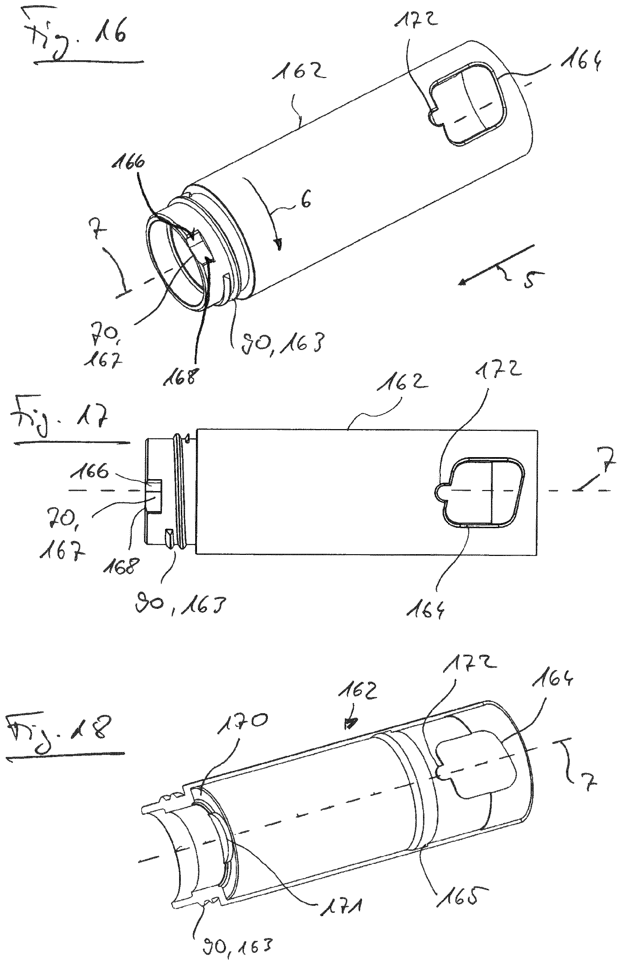

**Figs. 16** to **19** depict the outer housing 162 of the housing 160. The outer housing 162 is configured as a generally cylindrical part that extends parallel to the longitudinal axis 7. It is configured as a sleeve that is open at both longitudinal ends. Furthermore, it has a continuous bore that extends over its entire longitudinal length.

At the proximal end, the outer housing 162 comprises a second connection element 163 of the connector 90. The second connection element 163 is configured as a male connector and the first connection element 41 provided at the medicament holder 40 is configured as a female connector. Exemplarily, the second connection element 163 is configured as an outer thread and the first connection element 41 is configured as a corresponding inner thread.

A blocking mechanism 70 rotationally fixes the medicament holder 40 with respect to the housing 160 when the cap 10 is attached to the medicament holder 40. The blocking mechanism 70 comprises a first blocking element 50 that is provided at the medicament holder 40 and a second blocking element 167 that is provided at the housing 160, namely at the outer housing 162.

The first blocking element 50 is provided at an inner surface of the medicament holder 40 and the second blocking element 167 is provided at an outer surface of the outer housing 162. Exemplarily, the first blocking element 50 is configured as a radial protrusion that extends from the inner surface of the medicament holder 40 in the radial direction and the second blocking element 167 is configured as a radial depression within the outer surface of the outer housing 162. With other embodiments, the first blocking element 50 may instead be configured as the depression and the second blocking element 167 may be configured as the protrusion.

The first blocking element 50 extends parallel to the longitudinal axis 7 and has a length along the longitudinal axis 7 that is larger than a width perpendicular to the longitudinal axis 7. It thereby has a rounded outer surface that is rounded around an axis running parallel to the longitudinal axis 7. With the medicament holder 40, the first blocking element 50 is configured as a longitudinal cylindrical section.

The first blocking element 50 is located at a flexible element 52 and configured to flex in a radial direction perpendicular to the longitudinal axis 7. The flexible element 52 is formed within the outer wall of the medicament holder 40. It forms a free-standing structure that is surrounded by a gap 53. With a first end 51, the flexible element 52 is fixed to the medicament holder 40 while the first blocking element 50 is located at an opposing second end 53 of the flexible element 52. The second end 53 thereby is freestanding.

At the first end 51, the flexible element 52 comprises a flexing portion 56 that has a lower flexural stiffness than the remaining portion of the flexible element 52. The flexing portion 56 is configured as a section of the flexible element 52 that as a reduced thickness in the radial direction compared to the remaining portion of the flexible element 52. It comprises a circumferential groove provided on a surface of the flexible element 52. Exemplarily, the circumferential groove is provided at an outer surface of the flexible element 52. With other embodiments, the circumferential groove may also be provided at an inner surface of the flexible element 52.

The first blocking element 50 is located within a protruding axial section 43 of the medicament holder 40 that is connected to a recessed axial section 48 by a step 46. The recessed axial section 48 is located in the proximal direction 5 from the protruding axial section 43. Thereby, a radial extent of the protruding axial section 43 is larger than a radial extent of the recessed axial section 48 and also larger than a radial extent of the remaining part of the medicament holder 40 in the proximal direction 5.

The flexible element 52 extends from the recessed axial section 48 over the step 46 to the protruding axial section 43. It thereby comprises parts of the outer surfaces of the recessed axial section 48, the step 46, and the protruding axial section 43.

As can be seen from Figs. 16 and 17, the second blocking element 167 is located at a proximal end of the outer housing 162. It thereby is located in between the proximal end and the second connection element 163 of the connector 90. Furthermore, it is axially spaced from the second connection element 163 in the proximal direction 5.

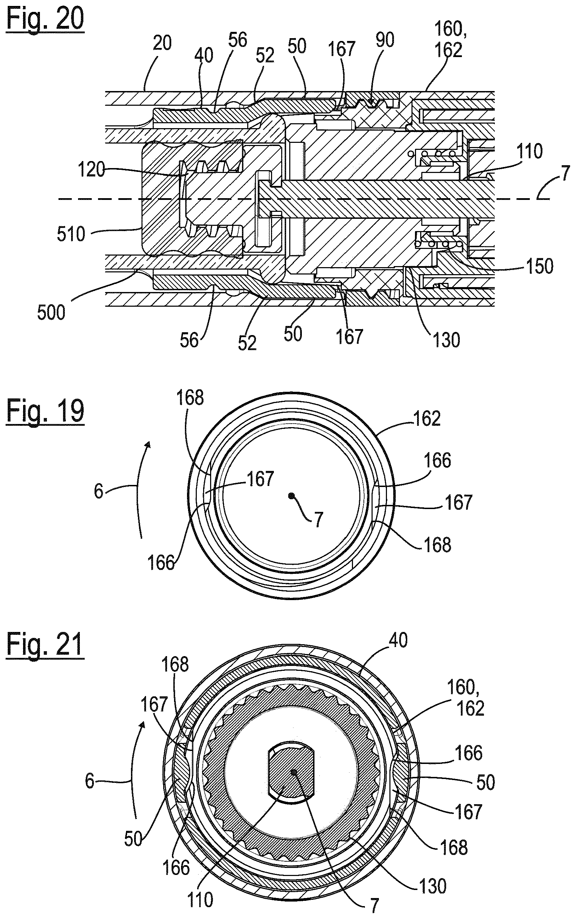

As can be seen from Fig. 19, the second blocking element 167 has a first side surface 166 and a second side surface 168. The second side surface 168 delimits the second blocking element 167 in a circumferential direction 6 and the first side surface 167 delimits the second blocking element 167 in a further circumferential direction opposite the circumferential direction 6. The first side surface 166 thereby is steeper than the second side surface 168. When the medicament holder 40 is being connected to the housing 160 of the dosing mechanism 100, the medicament holder 40 is rotated in the circumferential direction 6 with respect to the housing 160.

**Fig. 20** depicts a cross-sectional side view of the medicament holder 40 attached to the housing 160 and with the cap 20 attached to the medicament holder 40, shown in a cut plane parallel to the longitudinal axis 7. Furthermore, **Fig. 21** depicts a cross-sectional front view of the medicament holder 40 and the cap 20 in a plane perpendicular to the longitudinal axis 7.

The cap 20 interlocks the first blocking element 50 in engagement with the second blocking element 167 when the cap 20 is fully attached to the medicament holder 40. It thereby blocks the first blocking element 50 from bending away from the second blocking element 167 in the radial direction. The cap 20 engages with a surface of the first blocking element 50 that faces away from the second blocking element 167. The surface forms part of the flexible element 52 so that the attached cap 20 also engages with the flexible element 52.

An inner surface of the cap 20 thereby is shaped complementarily to the stepped portion of the outer surface of the medicament holder 40, the stepped portion comprising the step 46, at least parts of the protruding axial section 43 and at least parts of the recessed axial section 48.

As can be seen from Fig. 21, rotation of the medicament holder 40 with respect to the housing 160 in the further circumferential direction opposite the circumferential direction 6 rotates the first blocking element 50 against the second blocking element 167, namely against the first side surface 166. This engages the first blocking element 50 with the second blocking element 167 at the first side surface 166. With the cap 20 attached, the first blocking element 50 is interlocked and prevented from bending away from the second blocking element 167, thus rotationally locking the medicament holder 40 to the housing 160.

As further shown in Fig. 21, the blocking mechanism 70 comprises a further first blocking element 50 of the medicament holder 40 that is located opposite the first blocking element 50 and a further second blocking element 167 of the outer housing 162 that is located opposite the second blocking element 167. The first blocking elements 50 and the second blocking elements 167 are each identical in form and function.

The blocking mechanism 70 is configured to provide an audible and/or tactile feedback when the medicament holder 40 is being fully attached to the housing 160. Engagement of the first blocking elements 50 with the second blocking elements 167 thereby provide the feedback by producing a click. Likewise, the blocking mechanism 70 is configured to provide an audible and/or tactile feedback when the medicament holder 40 is moved out of its fully attached position with respect to the housing 160. This feedback is generated by the first blocking element 50 hitting the first side surface 166 of the second blocking element 167 when the medicament holder 40 is rotated out of its fully attached position.

Reverting to Figs. 1 to 5, the dosing mechanism 100 comprises an actuation unit 300 with a dose setting element 310 and a button 330 at its distal end 3. The dose setting element 310 has a generally ring-like shape outer surface that comprises a gripping structure 312. The dose setting element 310 is configured to be gripped by a user at the gripping structure 312 and to be rotated by the user around the longitudinal axis 7 of the medicament delivery device 1. Upon setting a dose, a corresponding marking 252 is shown in a window 164 in the outer housing 160 of the dosing mechanism 100. To reduce a set dose, the user rotates the dose setting element 310 back towards its zero position.

To deliver a set dose, the button 330 is configured to be pushed by the user in the proximal direction 5. This transfers the medicament delivery device 1 from a dose setting state into a dose delivery state.

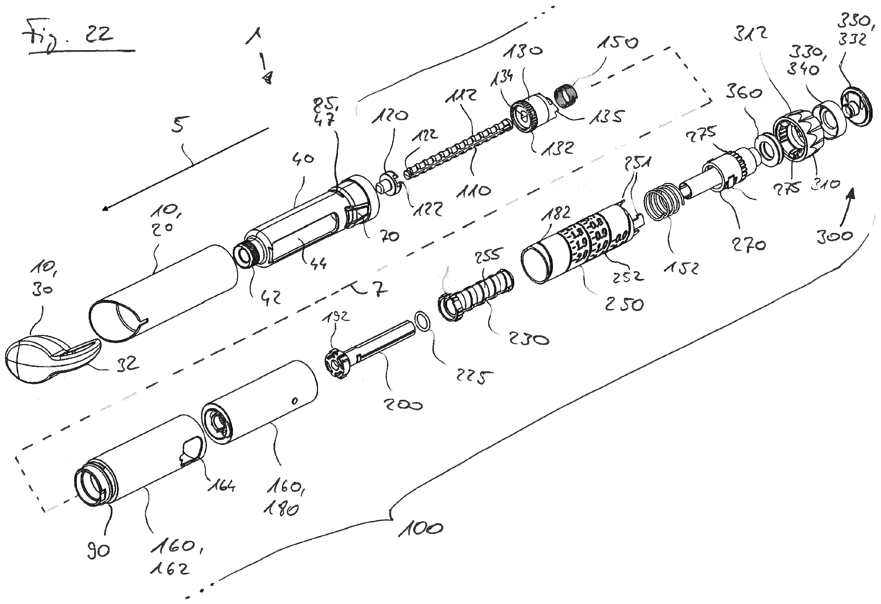

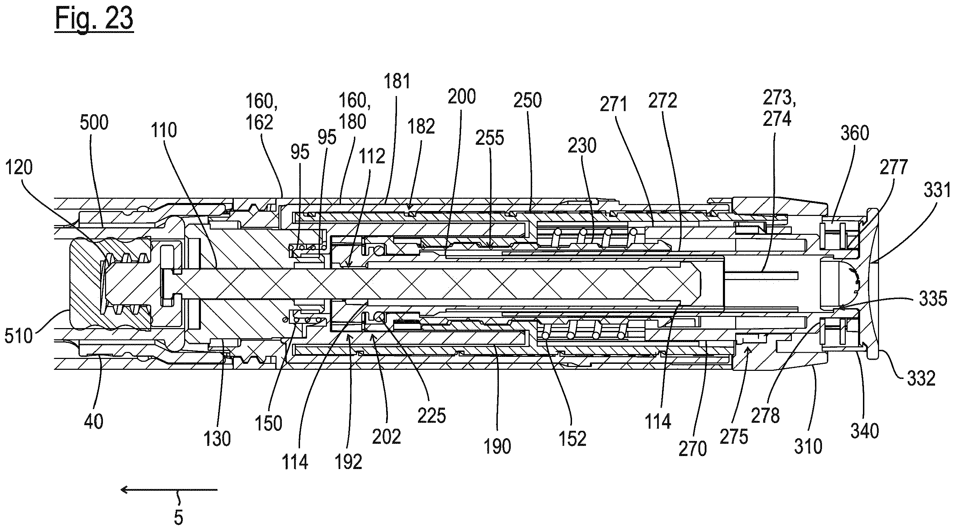

**Fig. 22** depicts an exploded perspective view of the medicament delivery device 1 and the dosing mechanism 100. **Fig. 23** depicts a cross-sectional side view of the dosing mechanism 100 in a plane parallel to the longitudinal axis 7, whereby the dosing mechanism 100 is in a dose setting state with no dose set.

The dosing mechanism 100 comprises a piston rod 110 that has a piston bearing 120 located at its proximal end. The piston rod 110 is configured to move into the medicament holder 40 and to push the piston 510 in the proximal direction 5 to deliver the medicament.

The piston rod 110 thereby pushes on the piston 503 via the piston bearing 120. The piston 503 has a blind hole at its distal end face and the piston bearing 120 comprises a pin at its front surface that extends into the blind hole of the piston 503. Furthermore, the blind hole of the piston 503 has a structured radial surface, namely a threaded radial surface. The piston 503 thereby is attached to the pin of the piston bearing 120 via a friction fit provided by the radial surface.

With the drug delivery device 1, the piston bearing 120 is configured as a part separate from the piston rod 110 and the piston bearing 120 is connected to the piston rod 110 by a connector. With other embodiments, the piston bearing 120 may also be formed integrally with the piston rod 110.

The working principle of the dosing mechanism 100 is generally described in document US20060258988A1. The disclosure of that working principle in document US20060258988A1 is incorporated into the present disclosure by reference in its entirety.

During dose setting and dose delivery, the piston rod 110 is rotationally fixed with respect to the housing 160. It is thereby guided within a piston rod guide 130 that is configured as a part separate from the housing 160 and that is rotationally fixed with respect to the housing 160 during both dose setting and dose delivery.

The piston rod 110 is permanently rotationally fixed with respect to the piston rod guide 130. The piston rod guide 130 has a central through hole 134 that receives the piston rod 110. The through hole 134 has a rotationally asymmetric radial cross-section and the outer surface of the piston rod 110 has a corresponding rotationally asymmetric radial cross-section. This provides an axial lock that rotationally fixes the piston rod 110 to the piston rod guide 130 while allowing relative axial movement between the piston rod 110 and the piston rod guide 130.

Besides the outer housing 162, the housing 160 comprises an inner housing 180 that is received within the outer housing 162. Exemplarily, the inner housing 180 is fully received within the outer housing 162.

While the outer housing 162 is configured as a metal part, such as a lathed metal part, the inner housing 180 is made from a plastic material, such as from a thermoplastic material. Furthermore, the inner housing 180 may be configured as an injection molded part. The inner housing 180 is permanently rotationally and axially fixed to the outer housing 162 so that the inner housing 180 and the outer housing 162 form a single functional component.

The piston rod guide 130 is connected to the inner housing 180 and may take up two axial positions with respect to the housing 160, a distal position and a proximal position. A first biasing member 150 acts between the housing 160 and the piston rod guide 130 and biases the piston rod guide 130 in the proximal direction 5 away from the housing 160 into the proximal position. As can be seen from Figures 6, 7 and 23, proximal movement of the piston rod guide 130 is limited by an axial stop 95 that comprises a first stop member at the inner housing 180 and a second stop member at the piston rod guide 130. The stop members engage with each other when the piston rod guide 130 reaches the proximal position and restrict further proximal movement of the piston rod guide 130. In the proximal position, the piston rod guide 130 is free to rotate with respect to the housing 160, while it is rotationally fixed with respect to the housing 160 in the distal position.

The piston rod guide 130 is in the proximal position when the medicament holder 40 comprising the medicament container 500 is removed from the housing 160. As can be seen from Figures 6, 7 and 23, the piston rod guide 130 is pushed in the distal direction by the medicament container 500 when the medicament holder 40 with the medicament container 500 is attached to the housing 160. This engages a locking member provided at a distal end of the piston rod guide 130 with a corresponding locking member of the inner housing 180 and rotationally locks the piston rod guide 132 the inner housing 180. As a consequence, the piston rod 110 is also rotationally locked with respect to the housing 160 as long as the medicament holder 40 with the medicament container 500 is attached to the housing 160.

The dosing mechanism 100 further comprises a nut 200 that is configured as a generally cylindrical hollow member and that at least partially surrounds the piston rod 110. A threaded connection 112 acts between the piston rod 110 and the nut 200. With the drug delivery device 1, the nut 200 is engaged with the piston rod 110 via the threaded connection 112. Generally speaking, the nut 200 forms a threaded part and the threaded connection 112 acts between the piston rod 110 and the threaded part.

The nut 200 is coupled to a connector 270 of the dosing mechanism 100 via a rotation lock 273. The nut 200 thereby is rotationally fixed and axially movable with respect to the connector 270. The connector 270 is axially fixed to the button 330 via an axial lock 335.

As can be seen from Figures 22 and 23, the button 330 comprises a button disc 332 and a button ring 340 that are axially fixed with respect to each other. With the drug delivery device 1, the button ring 340 is rotatable with respect to the button disc 332. With other embodiments, the button ring 340 may also be rotationally fixed with respect to the button disc 332. Alternatively, the button disc 332 and the button ring 340 may be configured as a single part.

The button disc 332 is located at the distal end 3 of the dosing mechanism 100 and the button ring 340 is attached on a proximal side of the button disc 332. The button ring 340 thereby surrounds the axial lock 335 provided between the button 330 and the connector 270.

A bearing 360 is located between the button 330 and the connector 270. The bearing 360 is configured as a pivot bearing with two opposing bearing surfaces that are orientated perpendicular to the longitudinal axis 7. The bearing 360 exemplarily comprises a ball bearing and the bearing surfaces are part of two washers that are located at either side of the ball bearing along the longitudinal axis 7. The bearing 360 is configured to facilitate rotation of the connector 270 with respect to the button 330 when the button 330 is pushed in the proximal direction against the connector 270.

As can be seen from Fig. 23, the bearing 360 surrounds a distal cylindrical part 277 of the connector 270. In the proximal direction, it axially rests on a shoulder 278 provided at the outside surface of the distal cylindrical part 277. The button ring 340 radially surrounds the bearing 360. A proximal inner surface of the button ring 340 rests against the distal part of the bearing 360, namely against the distal washer of the bearing 360.

The dosing mechanism 100 further comprises a dose member 250 that is configured as a generally cylindrical hollow member. The dose member 250 surrounds the nut 200 and the piston rod 110. Furthermore, an outer surface of the dose member 250 is located directly at an inner surface of the housing 160, namely at an inner surface of the inner housing 180.

A threaded connection 182 acts between the dose member 250 and the housing 160. Exemplarily, the dose member 250 engages the housing 160 via the threaded connection 182. The threaded connection 182 comprises an outer thread on an outer surface of the dose member 250 that engages with an inner thread on an inner surface of the inner housing 180. The dose member 250 forms a threaded part and the threaded connection 182 acts between the threaded part and the housing 160.

The dose member 250 is permanently rotationally and axially fixed with respect to the dose setting element 310 by a connector 251. The connector 251 is configured as a snap-fit connector.

The dosing mechanism 100 further comprises a driver 230 that is configured as a generally cylindrical hollow member and that at least partially surrounds the nut 200. Furthermore, the driver 230 is located radially in between the nut 200 and the dose member 250.

A threaded connection 255 acts between the driver 230 and the dose member 250. The driver 230 thereby is threadedly engaged with the dose member 250 via the threaded connection 255. The threaded connection 255 comprises an outer thread on an outer cylindrical surface of the driver 230 that engages with an inner thread on an inner cylindrical surface of the dose member 250. Furthermore, the driver 230 is rotationally fixed to the housing 160 via a rotation lock 202. The rotation lock 202 allows longitudinal movement of the driver 230 with respect to the housing 160.

The dosing mechanism 100 further has a clutch 275 that acts between the nut 200 and the dose member 250. The clutch 275 rotationally locks the nut 200 to the dose member 250 in a closed state and allows relative rotation between the nut 200 and the dose member 250 in an opened state. During dose setting, the clutch 275 rotationally locks the nut 200 to the dose member 250 and, during dose delivery, the clutch 270 rotationally decouples the nut 200 from the dose member 250.

The clutch 275 is exemplarily provided between the connector 270 and the dose setting element 310. It comprises radial teeth on an outer surface of the connector 270 that engage with corresponding radial teeth on an inner surface of the dose setting element 310 when the clutch 275 in the closed state. The clutch 275 is configured to transfer from the closed state into the opened state by axially moving the connector 270 with respect to the dose setting element 310. During dose setting, the clutch 275 is biased into its closed state. A second biasing member 152 acts between the connector 270 and the dose setting element 310 and biases the connector 270 in the distal direction with respect to the dose setting element 310. Like with the first biasing element 150, the second biasing element 152 is configured as a compression spring. The second biasing element 310 is provided in between the dose member 250 and the connector 270 and engages with both the dose member 250 and the connector 270.

Proximal movement of the button 330 moves the connector 270 in the proximal direction 5 against the force of the second biasing member 152. This transfers the clutch 275 from its closed state into its opened state.

With alternative embodiments, the dose setting element 310 may also be rotationally and axially fixed with respect to the button 330. The button 330 and the dose setting element 310 then may be permanently axially and rotationally fixed to the connector 270 instead of the connection via the axial lock 335. Alternatively, the dose setting element 310 and button 330 may be connected to the connector 270 via the axial lock 335. The dosing mechanism 100 then may comprise an additional clutch that rotationally locks the dose setting element 310 and the button 330 to the dose member 250 during dose setting and rotationally decouples the dose setting element 310 and the bottom 330 from the dose member 250 during dose delivery. Like the clutch 275, the additional clutch may be transferred into its opened state by pushing the button 330 in the proximal direction 5. The clutch 275 then may act between the dose member 250 and the connector 270, but not between, on the one hand, the button 330 and the dose setting member 310 and, on the other hand, the dose member 250 and the connector 270.

During dose setting, both the nut 200 and the dose member 250 are rotationally fixed to the dose setting element 310. Rotation of the dose setting element 310 then causes the nut 200 and the dose member 250 to move axially with respect to the piston rod 110 and the housing 160 due to, on the one hand, the threaded connection 112 between the nut 200 and the piston rod 110 and, on the other hand, the threaded connection 182 between the dose member 250 and the housing 160. Upon increasing a set dose, the nut 200 and the dose member 250 both move axially in the distal direction. A set dose may be decreased by rotating the dose setting element 310 in an opposite direction as during dose setting.

A pitch of the threaded connection 112 is different from a pitch of the threaded connection 182 so that the nut 200 and the dose member 250 travel differing axial distances upon rotation by the same angle. With the drug delivery device 1, the pitch of the threaded connection 112 is smaller than the pitch of the threaded connection 182 and the dose member 250 travels a larger axial distance than the nut 200.

A pitch of the threaded connection 255 acting between the rotationally fixed driver 230 and the dose member 250 is adapted to cause the driver 230 to axially move together with the nut 200 along the longitudinal axis 7 upon rotation of the dose setting element 310 during dose setting. The pitch of the threaded connection 255 thereby is at most a difference between a maximum pitch of the threaded connection 112 and a minimum pitch of the threaded connection 182. The maximum pitch of the threaded connection 112 thereby is given by an upper limit of fabrication tolerances of the threaded connection 112 and the minimum pitch of the threaded connection 182 is given by a lower limit of fabrication tolerances of the threaded connection 182. This prevents the nut 200 from advancing faster in the distal direction than the driver 230 and avoids blocking movement of the nut 200 by the driver 230.

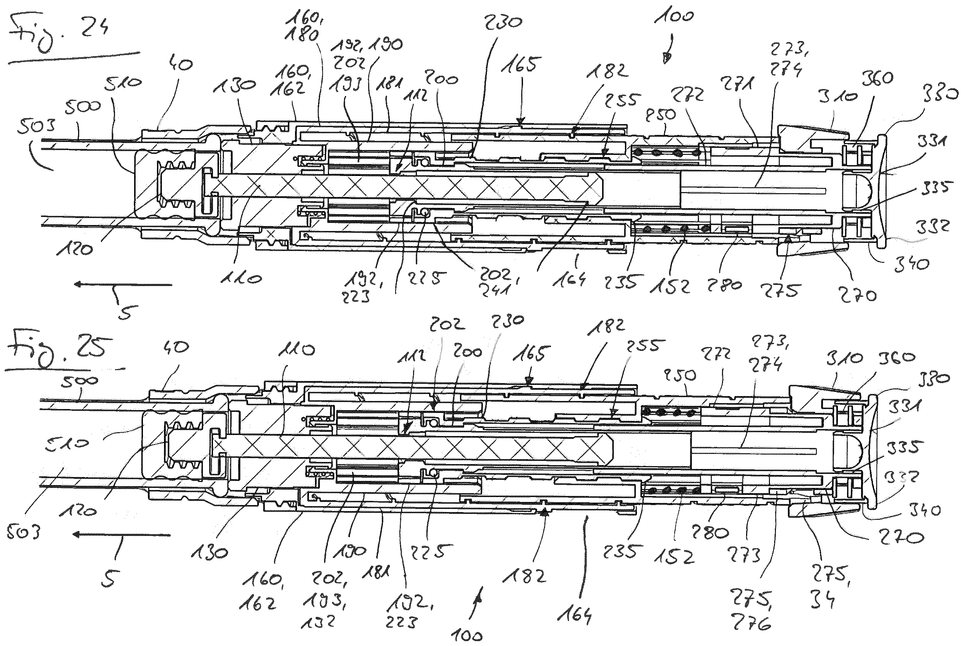

**Fig. 24** depicts a cross-sectional side view of the dosing mechanism 100 after having set a maximum dose. The dose member 250 has been screwed out of the housing 160 in the distal direction and the dose setting element 310 and the button 330 have moved together with the dose member 250 in the distal direction. The nut 200 has been screwed along the piston rod 110 in the distal direction and the driver 230 has translated together with the nut 200 in the distal direction without rotation. To set the maximum dose, the nut 200 and the dose member 250 have performed at least one, namely at least two full revolutions around the longitudinal axis 7. Exemplarily, the maximum dose is set by rotating the nut 200 and the dose member 250 by three full revolutions around the longitudinal axis 7.

A latching mechanism 192 acts between the nut 200 and the housing 160. The nut 200 thereby rotationally couples the dose setting element 310 and the dose member 250 to the latching mechanism 192. The latching mechanism 192 also acts as a dose definition mechanism that defines discrete rotational positions of the nut 200 and the dose setting element 310 and the dose member 250, wherein the discrete rotational positions correspond to settable doses.

The latching mechanism 192 comprises a first latching part 223 that is rotationally fixed to the nut 200 and a second latching part 193 that is rotationally fixed to the housing 160. One of the first and second latching part 223, 193, namely the second latching part 193, comprises longitudinal webs that are circumferentially distributed around the longitudinal axis 7 and the other one of the first and second latching part 123, 193, namely the first latching part 223, comprises a flexible element engaging with the longitudinal webs upon rotation of the first latching part 223 with respect to the second latching part 193. The first latching part 223 is additionally axially fixed to the nut 200 and the second latching part 193 is axially fixed to the housing 160.

With the dosing mechanism 100, the nut 200 engages the housing 160 via the latching mechanism 192. The first latching part 223 is integrally formed with the nut 200 and the second latching part 193 is integrally formed with the housing 160, namely with the inner housing 180. The second latching part 193 is thereby provided at an inner section 190 of the inner housing 180. The inner section 190 comprises the second latching part 193 on a cylindrical inner surface.

The inner section 190 is configured as a hollow cylindrical portion of the inner housing 180 and extends from a proximal end of the inner housing 180 in the distal direction. The inner section 190 is surrounded by an outer wall of the inner housing 180, whereby the outer wall comprises the threaded connection 182 to the dose member 250. During dose setting, the dose member 250 moves within an annular space in between the outer wall of the inner housing 180 and the inner section 190. The inner section 190 has an open distal end and the nut 200 and the driver 230 extend through the open distal end.

Relative rotation of the nut 200 with respect to the housing 160 during dose setting causes the first latching part 223 to rotate with respect to the second latching part 193 and to thereby provide an audible and/or tactile feedback to the user. The feedback comprises a click each time a dose has been set. The feedback is produced by the first latching part 223 engaging with constitutive grooves in between the webs of the second latching part 193.

The rotation lock 202 that rotationally fixes the driver 230 to the housing 160 comprises at least one first lock element 241 that is rotationally fixed to the driver 230 and at least one second lock element that is rotationally fixed to the housing 160. The rotation lock 202 is configured as a splined connection that extends along the longitudinal axis 7. One of the first lock element 241 and the second lock element is configured as a longitudinal groove and the other one of the first lock element 241 and the second lock element is configured as a corresponding longitudinal ridge engaging with the longitudinal groove.

With the dosing mechanism 100, the at least one second lock element is provided by the second latching part 193 of the latching mechanism 192. The second lock element is thereby formed by a longitudinal groove between two of the longitudinal webs of the second latching part 193.

An end stop 235 prevents setting of a dose that exceeds the maximum dose. The end stop 235 acts between the dose member 250 and the driver 230. The end stop 235 limits axial movement of the driver 230 with respect to the dose member 250. It is configured as an axial stop and stop surfaces of the end stop 235 engage by axially moving towards each other. The end stop 235 is located at a distal end of the threaded connection 255.

The end stop 235 comprises a radial protrusion on the outer surface of the driver 230 that engages with a radial step formed at an inner surface of the dose member 250. The radial step on the inner surface of the dose member 250 is formed at a distal end of an inner sleeve of the dose member 250, whereby the inner sleeve carries the threaded connection 255 to the driver 230.

After having set the dose, the dosing mechanism 100 is configured to transfer from the dose setting state to a dose delivery state upon a user pushing the button 330 in the proximal direction 5. **Fig. 25** depicts the dosing mechanism 100 in the dose delivery state prior to delivering the maximum dose.

The button 330 has moved in the proximal direction 5 with respect to the dose setting element 310 until the button 330 axially engages with the dose setting element 310 via the bearing 360. The bearing 360 thereby rests against in inner distal surface of the dose setting element 310. The bearing 360 and the button ring 340 surrounding the bearing 360 are received in a distal open cavity of the dose setting element 310.

Proximal movement of the button 330 also moves the connector 270 in the proximal direction 5 with respect to the dose setting element 310 via the axial lock 335. This opens the clutch 275 so that the dose member 250 and the dose setting element 310 are allowed to rotate with respect to the connector 270 and the nut 200.

Further proximal movement of the button 330 then pushes the dose member 250 in the proximal direction 5 and the proximal force exerted on the button 330 is transferred to the dose member 250 via the bearing 360. Due to the threaded connection 182, the dose member 250 rotates back into the housing 160. Proximal movement and rotation of the dose member 250 also causes proximal movement of the driver 230 via the threaded connection 255. Since the driver 230 is prevented from rotating by the rotation lock 202 it is screwed in the distal direction with respect to the dose member 250 due to the threaded connection 255.

The driver 230 is axially coupled to the nut 200 in the proximal direction 5 and configured to push the nut 200 in the proximal direction 5, as it is further detailed below. Since the nut 200 is rotationally decoupled from the dose member 250 when the clutch 275 is in its opened state, it does not receive a torque from the rotating dose member 250. Rotation of the dose member 250 with respect to the nut 200 is further facilitated by the axial lock 335 that allows rotation between the connector 270 and the button 330 and the bearing 360.

The latching mechanism 192 acts as a rotation break during dose delivery that prevents the nut 200 from rotating with respect to the piston rod 110. This locks the threaded connection 112 between the nut 200 and the piston rod 110 so that the piston rod 110 is forced to move axially together with the nut 200 in the proximal direction 5. The piston rod 110 then moves into the medicament holder 40 and the medicament container 500 and advances the piston 510 in the proximal direction 5 to expel the medicament from the medicament compartment 503.

The dosing mechanism 100 comprises a zero dose stop that stops axial movement of the piston rod 110 in the proximal direction 5 at the end of dose delivery. The zero dose stop acts between the dose member 250 and the housing 160. It limits proximal movement of the dose member 250 with respect to the housing 160. It is configured as an axial stop with stop elements that engage with each other by moving towards each other along the longitudinal axis 7. The stop elements comprise stop surfaces that are orientated generally perpendicular, such as perpendicular, to the longitudinal axis 7.

The zero dose stop is formed at the dose setting element 310 and the housing 160, exemplarily the outer housing 162. A first stop element is thereby formed at the dose setting element 310 and a second stop element is formed at the housing 160. The first stop element is provided by the proximal end surface of the dose setting element 310 and the second stop element is provided by the distal end surface of the housing 160, exemplarily at the outer housing 162, namely by the distal end surface of the outer housing 162. As can be seen from Fig. 23, the proximal end surface of the dose setting element 310 and the distal end surface of the inner housing 180 are spaced apart from each other in the zero dose state.

With alternative embodiments, the second stop element may also be formed at the inner housing 180. For example, the second stop element may be formed by a distal end surface of the inner housing 180.

The dosing mechanism 100 further comprises a last dose stop 114 that prevents setting of a dose that is larger than a remaining amount of medicament in the medicament compartment 503. The last dose stop 114 acts between the piston rod 110 and the nut 200. It comprises a radial protrusion on the outer surface of the piston rod 110, the radial protrusion being located at the distal end of the outer thread of the threaded connection 112 between the piston rod 110 and the nut 200. The last dose stop 114 further comprises a radial step on the inner surface of the nut 200, whereby the step is provided at the distal end of the inner thread of the threaded connection 112. The step protrudes radially inwardly and engages with the protrusion on the distal end of the piston rod 110 when the nut 200 reaches the distal end of the piston rod 110 during dose setting. With each dose that is delivered, a zero dose position of the nut 200 with respect to the piston rod 110 sequentially moves in the proximal direction 5 along the piston rod 110. A length of the outer thread on the piston rod 110 is adapted to cause engagement of the last dose stop 114 and to stop distal movement of the nut 200 when a set dose equals the remaining dose in the medicament compartment 503.

With alternative embodiments of the dosing mechanism 1, the pitch of the threaded connection 112 between the piston rod 110 and the nut 200 may also be larger than the pitch of the threaded connection 182 between the housing 160 and the dose member 250. Alternatively, the pitch of the threaded connection 112 may also equal the pitch of the threaded connection 182. With such an embodiment, the driver 230 may be connected to the dose member 250 by an axial lock that axially fixes the driver 230 to the dose member 250 while allowing relative rotation of the driver 230 with respect to the dose member 250.

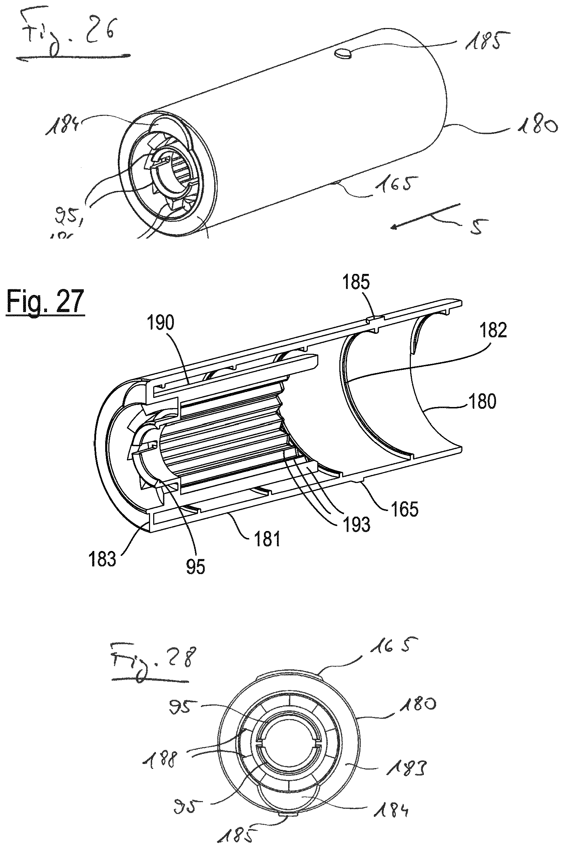

**Figs. 26** to **28** depict the inner housing 180. The inner housing 180 is formed as a generally cylindrical hollow part. It comprises an outer section 181 that is integrally formed with the inner section 190. The outer section 181 forms an outer wall of the inner housing 180. An inner surface of the inner section 190 comprises the inner thread of the threaded connection 182. The inner thread thereby extends over the complete length of the inner surface along the longitudinal axis 7.

On an outer wall of the outer section 181, the inner housing 180 comprises a locking element of a first axial lock 165 that axially locks the inner housing 180 with respect to the outer housing 162 in the distal direction. The axial lock 165 is configured as a snap-lock that allows insertion of the inner housing 180 into the outer housing 162 in the proximal direction 5 and blocks distal movement of the inner housing 180 out of the outer housing 162 in the distal direction after engagement. The locking element of the inner housing 180 thereby engages with a corresponding locking element provided on an inside surface of the outer housing 162, see Fig. 18.

The locking element of the inner housing 180 thereby is configured as a radial protrusion on the outer surface of the inner housing 180. The protrusion has a radial surface that is orientated parallel to a radial plane perpendicular to the longitudinal axis 7 at its distal end and a beveled surface at its proximal end to facilitate insertion of the inner housing 180 into the outer housing 162.

The locking element of the outer housing 162 is configured as a circumferential groove with a radial end surface in the distal direction that is perpendicular to the longitudinal axis 7. The groove thereby covers the entire circumference of the inner surface of the outer housing 162.

Movement of the inner housing 180 with respect to the outer housing 162 in the proximal direction is restricted by an axial stop. The axial stop is formed between a proximal end surface 183 of the inner housing 180 and a distally facing proximal end surface 170 of the outer housing 162. The end surface 170 thereby delimits an interior cavity that receives the inner housing 180 in the proximal direction 5.

A first rotational lock between the inner housing 180 and the outer housing 162 is provided by a protrusion 184 at the proximal end surface 183 of the inner housing 180 and a corresponding recess 171 at the end surface 170 of the outer housing 162. The protrusion 184 and the recess 171 are thereby rotationally asymmetric with respect to the longitudinal axis 7.

A second rotational lock between the inner housing 180 and the outer housing 162 is provided by a protrusion 185 at the outer surface of the inner housing 180 that engages with a corresponding recess 172 provided at the inner surface of the outer housing 162. The recess 172 thereby is configured as a notch at a proximal edge of the window 164. The inner housing 180 is made from a transparent material and covers the window 164 from the inside. The protrusion 185 then radially protrudes into the recess 172 of the window 164.

At the proximal end, the inner housing 180 comprises first stop members 186 of the axial stop 95 that limits axial movement of the piston rod guide 130 in the proximal direction 5. The first stop members 186 are configured as flexible snap hooks that extend from the inner housing 180 in the proximal direction 5. The snap hooks have a cylindrical shape.

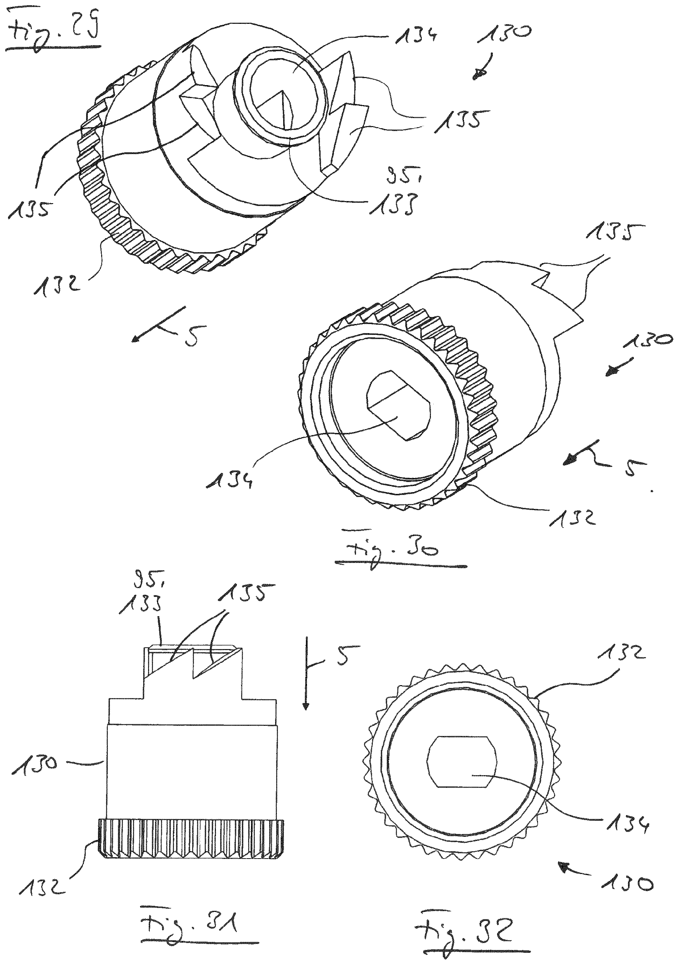

**Figs. 29** to **32** depict the piston rod guide 130. At its distal end, the piston rod guide 130 has a second stop member 133 of the axial stop 95 that is configured to engage with the first stop members 186. The second stop member 133 is configured as a cylindrical protrusion that extends from the axial stop 95 in the distal direction. It is placed radially inward from the first stop members 186. The snap hooks formed by the first stop members 186 then engage with a radial outer rim provided at a distal end of the second stop member 133 when the piston rod guide 130 is in its proximal position with respect to the housing 160.

To rotationally lock the piston rod guide 130 in its distal position with respect to the housing 160, the inner housing 180 comprises a first locking member 188 of a rotation lock that acts between the housing 160 and the piston rod guide 130. The first locking member 188 is configured as a longitudinally toothed radial surface at the proximal end of the inner housing 180. The first locking member 188 thereby surround the axial lock 95.

The piston rod guide 130 comprises a second locking member 135 of the rotation lock. The second locking member 135 comprises longitudinal teeth that are configured to engage with the toothed circumferential section of the inner housing 180. The teeth thereby extend from the piston rod guide 130 in the distal direction. They are placed radially outwards from the second stop member 133.

The dosing mechanism 110 comprises a re-setting member that is configured to move the piston rod 110 back into the housing 160 after the medicament holder 40 has been detached from the housing 160. The resetting member is formed by the piston rod guide 130. A rotation of the piston rod guide 130 with respect to the housing 160 when the medicament holder 40 is detached from the housing 160 causes the piston rod 110 to move relative to the housing 160.

Since the piston rod 110 is rotationally fixed to the piston rod guide 130, the rotation of the piston rod guide 130 forces the piston rod 110 to also rotate. This screws the piston rod 110 along the threaded connection 182 between the piston rod 110 and the nut 200. The user then may reset the dosing mechanism 100 by rotating the piston rod guide 130 when the piston rod guide 130 is in its proximal position. To facilitate rotation of the piston rod guide 130, the piston rod guide 130 comprises a gripping structure 132 on its outer circumference. The gripping structure 132 thereby is located at a proximal end of the piston rod guide 130.

In addition, the threaded connection 182 acting between the piston rod 110 and the nut 200 may be configured as a non-self-locking threaded connection. The piston rod 110 then may be forced to helically move along the threaded connection 182 by pushing it towards the housing 160. This allows a user to reset the dosing mechanism 100 by manually pushing the piston rod 110 back into the housing 160. The movement of the piston rod 110 then forces the piston rod guide 130 to rotate with respect to the housing 160.

The dosing mechanism 100 is an exemplary dosing mechanism that has a first member that is configured to rotate with respect to a second member around the longitudinal axis 7 during dose setting and that is configured to transfer an axial force to the second member in the proximal direction 5 during dose delivery. The first member thereby is formed by the driver 230 and the second member is formed by the nut 200.

In general, the present disclosure relates, inter alia, at dosing mechanisms having such first and second members and in which a friction break is provided in between the first member and the second member to provide a rotation break between the first member and the second member when the first member transfers a force to the second member.

With the dosing mechanism 100, such a friction break is formed by a friction brake 225. The friction break 225 is exemplarily configured as a O-ring that sits on an outer surface 206 of the nut 200.

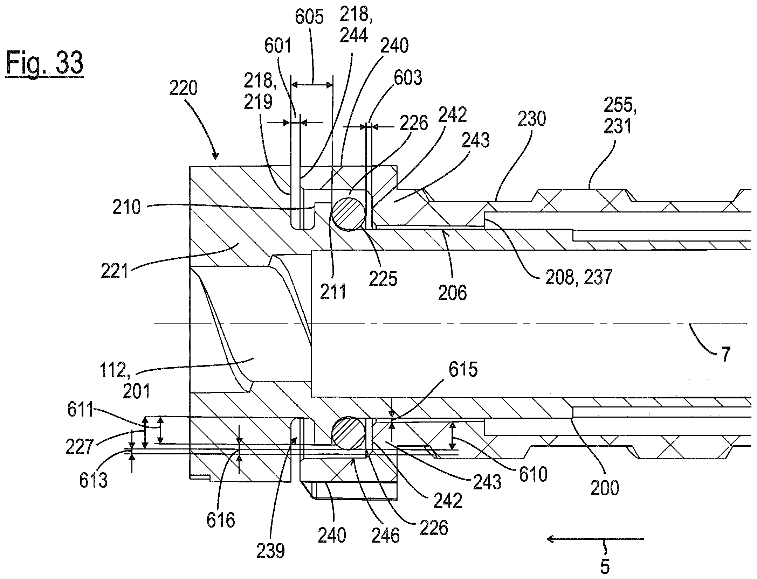

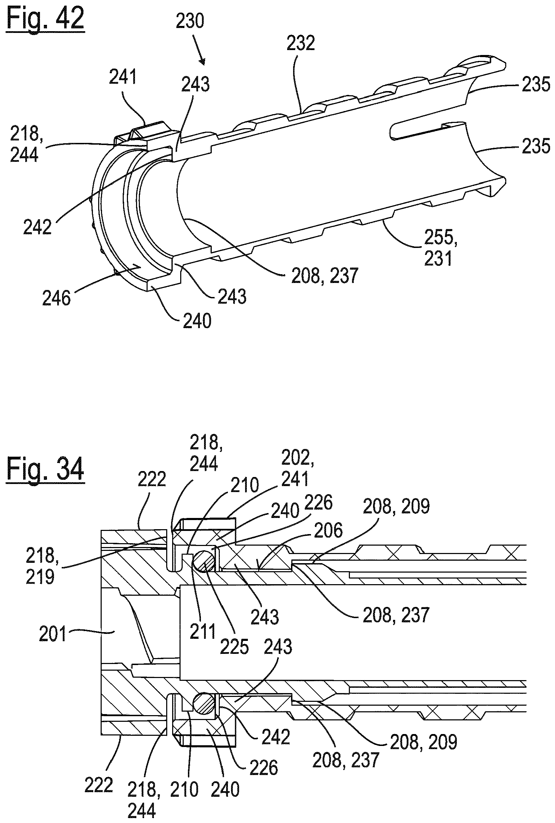

**Fig. 33** depicts a cross-sectional side view in a cut plane parallel to the longitudinal axis 7 showing the nut 200 and the driver 230 with the friction brake 225 positioned in between and **Fig. 34** depicts a corresponding cross-sectional side view in a cut plane parallel to the longitudinal axis 7 and perpendicular to the cut plane of Fig. 33.

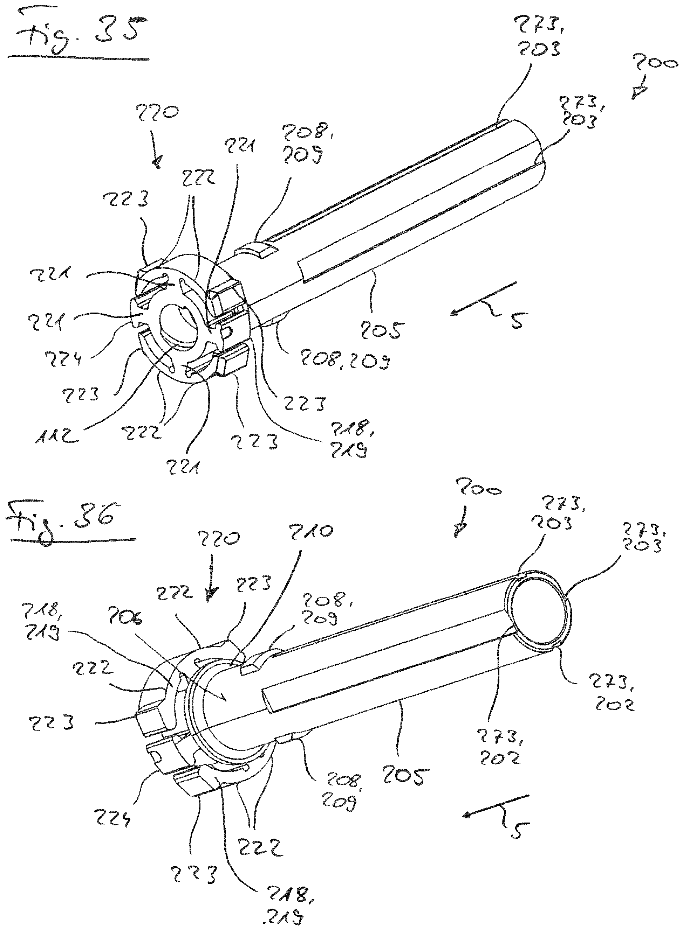

The nut 200, which is also shown in **Figs. 35** to **39****,** comprises a generally cylindrical body 205 and a protruding part 220 that radially protrudes from the body 205. The protruding part 220 is exemplarily located at a proximal end of the body 205. Within a central bore of the protruding part 220, the nut 200 comprises an inner thread 201 of the threaded connection 112 to the piston rod 110.

The protruding part 120 comprises the first latching part 223 of the latching mechanism 192 on its outer circumferential surface. The first latching part 223 comprises a longitudinal protrusion located at a free end of a flexible arm. The flexible arm extends in the circumferential direction and has a fixed end that is fixed to a bar 221 that radially extends from the body 205. The first latching part 223 is configured to flex in the radial direction perpendicular to the longitudinal axis 7. It furthermore is configured to bend radially inward upon disengaging from the second latching part 193 of the inner housing 180.

In total, the nut 200 comprises four of the first latching parts 223. The first latching parts 223 are located pairwise opposite each other with respect to the longitudinal axis 7. Furthermore, each first latching part 223 is attached together with a neighboring first latching part 223 to a common bar 221. The nut 200 thereby comprises two of the bars 221 located at opposite positions with respect to the longitudinal axis 7. In between two first latching parts 223 that are connected to different ones of the bars 221, the nut 200 comprises two rigid arms 224 each of which is connected by an additional one of the bars 221 to the body 205.

Outer circumferential surfaces of the flexible arms 222 and outer circumferential surfaces of the rigid arms 224 are configured as cylindrical surfaces around the longitudinal axis 7. The circumferential surfaces of the flexible arms 222 and the circumferential surfaces of the rigid arms 225 thereby have the same radius.

In the distal direction from the protruding part 220, the nut 200 comprises a contact structure 210. The contact structure 210 is spaced apart from the protruding part 220 in the distal direction. It thereby is separated from the protruding part 220 by a gap.

The contact structure 210 is configured as a radial protrusion extending from the outer surface 206 of the body 205. It forms a ring-like structure that circumferentially fully encloses the outer surface 206 of the body 205.

The contact structure 210 is located on a proximal side of the friction brake 225. It is configured to limit proximal movement of the friction brake 225. The contact structure 210 comprises a first contact section 211 that is configured to contact the friction brake 225. Furthermore, the first contact section 211 is configured to stop movement of the friction brake 225 in the proximal direction 5.

The first contact section 211 is configured as a contact surface. The first contact section 211 has a shape that is adapted to an outer shape of the proximal portion of the friction brake 225. The shape is exemplarily formed as a rounded transition from the outer surface 206 of the body 205 to a radial surface portion of the first contact section 211 that is perpendicular to the longitudinal axis 7.

The first contact section 211 circumferentially fully surrounds the longitudinal axis 7. Furthermore, the first contact section 211 is configured to contact the friction brake 225 continuously around the longitudinal axis 7.

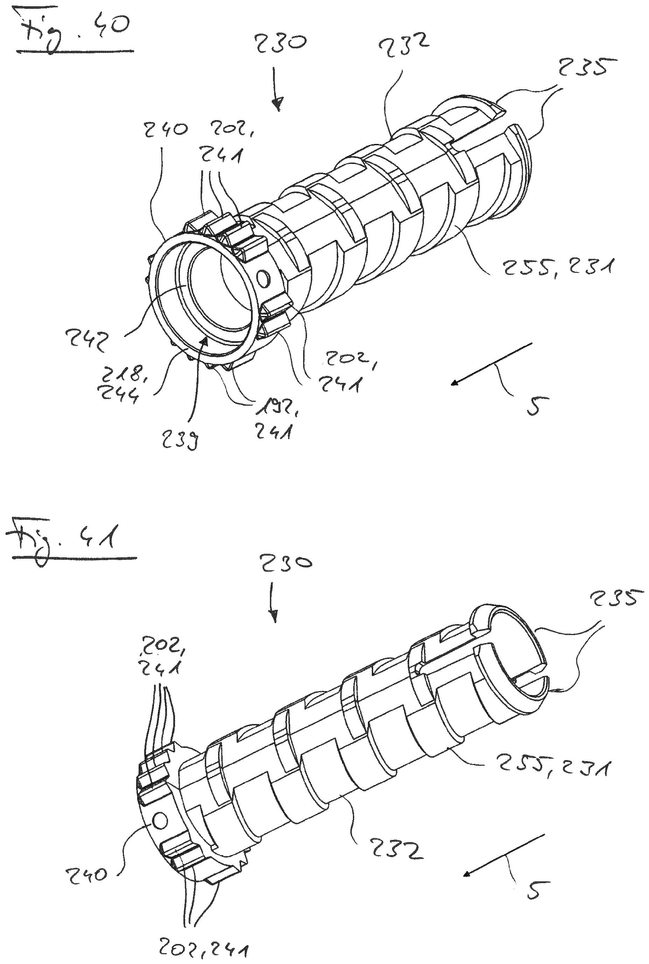

The driver 230, which is shown in detail in **Figs. 40** to **42****,** radially surrounds the body 205 of the nut 200. The driver 230 comprises a body 232 that is configured as a hollow cylindrical member. An outer thread 231 of the threaded connection 255 to the dose member 250 is provided on an outer circumferential surface of the body 232. At the distal end of the body 232, the driver 230 comprises the radial protrusion of the end stop 235.

The driver 230 further comprises a protruding part 240 that radially protrudes from the body 230. The protruding part 240 is located at a proximal end of the driver 230.

At its outer circumferential surface, the protruding part 240 comprises the at least one first lock element 241 of the rotation lock 202 between the driver 230 and the housing 160. The first lock element 241 is configured as a radial tooth extending radially from the outer surface of the driver 240 and being elongated along the longitudinal axis 7. As can be seen from Figs. 40 and 41, the driver 230 comprises several of the first lock element 241 that are distributed around the circumference of the driver 230.

The protruding part 240 circumferentially surrounds the friction brake 225 and the contact section 211 of the nut 200 in the radial direction. It comprises an open end cavity 239 at the proximal end of the protruding part 240. The protruding part 240 thereby extends in the proximal direction 5 over the friction brake 225 and the contact section 211 towards the protruding part 220 of the nut 200.

The driver 230 comprises a second contact section 242 that is located in the distal direction from the friction brake 225 along the longitudinal axis 7. The first contact section 211 of the nut 200, the friction brake 225 and the second contact section 242 thereby are axially aligned with each other and axially overlap with each other parallel to the longitudinal axis 7.

The second contact section 242 is circumferentially closed around the longitudinal axis 7. It forms a ring-like structure surrounding the longitudinal axis 7. The second contact section 242 is exemplarily formed as a contact surface. The contact surface is orientated perpendicular to the longitudinal axis 7.

The second contact section 242 is located at a distal end of the end cavity 239. It forms an inwardly directed circumferential shoulder on an inner circumferential surface of the end cavity 239. The second contact section 242 is part of a second contact structure 243 that is formed by the protruding part 240 of the driver 230.

The second contact section 242 is configured to push against the friction brake 225 when the driver 230 is moved in the proximal direction 5 during dose delivery. This compresses the friction brake 225 between the first contact section 211 and the second contact section 242.

A hard stop 218 is provided between the driver 230 and the nut 200. The hard stop 218 comprises a first stop part 244 and a second stop part 219 that engage with each other when the friction break 235 is compressed to a predefined maximum compression. The first stop part 244 is provided at the driver 230, namely at the protruding part 240 of the driver 230, and the second stop part 219 is provided at the nut 200, namely at the protruding part 220 of the nut 200. The first stop part 144 is formed by a proximal end surface of the driver 230. The end surface forms an end surface of an outer wall of the end cavity 239. The end surface provides a first stop surface of the hard stop 218.

The second stop part 219 is formed by a distal facing surface of the nut 200. The distal facing surface thereby forms a distal facing end surface of the protruding part 220. The distal facing surface provides a second stop surface of the hard stop 218.

Figures 33 and 34 show the driver 230 and the nut 200 in a relaxed state when no axial force is applied to the driver 230. Furthermore, the driver 230 is positioned in its most distal position with respect to the nut 200. This most distal position is defined by a further stop 208 that acts between the nut 200 and the driver 240 and limits axial movement of the driver 230 in the distal direction with respect to the nut 200. The further stop 208 comprises a first stop element 209 provided at the outer surface 206 of the nut 200 and a second stop element 237 provided on an inner surface of the driver 230. The first and second stop element 209, 237 engage with each other to limit the axial movement of the driver 230 in the distal direction with respect to the nut 200.

The first stop element 209 is configured as a radial protrusion on the outer surface 206 of the nut 200 and the second stop element 237 is configured as a radial protrusion on an inner surface of the driver 230. The first stop element 209 has an angled distal surface that is configured to allow relative movement of the driver 230 in the proximal direction 5 with respect to the nut 200 during assembly of the dosing mechanism 100. A proximal surface of the first stop element 209 is configured as a radial surface perpendicular to the longitudinal axis 7. It prevents detachment of the driver 230 from the nut 200 after assembly.

In the relaxed state shown in Figures 33 and 34, the second contact section 242 has an axial distance 603 from the friction brake 225. Furthermore, the first stop part 244 of the hard stop 218 has a distance 601 from the second stop part 219. The distance 601 thereby is larger than the distance 603. The difference between the distance 601 and the distance 603 defines the maximum compression of the friction brake 225.

The friction brake 225 is located within a cavity 226 that is radially bounded by the outer surface 206 of the nut 200 and an inner surface 246 of the end cavity 239 and that is axially bounded by the first and second contact sections 211, 242. A first radial spacing 615 between the nut 200 and the driver 230 at the distal end of the cavity 226 is smaller than the radial height 227 of the friction brake 225 from the nut 200 perpendicular to the longitudinal axis 7. Likewise, a second radial spacing 616 between the driver 230 and the nut 200 at the proximal end of the cavity 226 is smaller than the radial height 227 of the friction brake 225 from the nut 200.

The first contact section 211 overlaps with the friction brake 225 in the radial direction over a first radial height 611. Furthermore, the second contact section 242 overlaps with the friction brake 225 over a second radial height 610.

The inner surface 246 of the driver 230 has a clearance 613 from the friction brake 225. The clearance 613 may be large enough to prevent the friction brake 225 from touching the inner surface 246 when it has the predefined maximum compression.

As can be seen from Figs. 33 and 34, the nut 200 does not axially overlap with the friction brake 225 in between the friction brake 225 and the second contact section 242 of the driver 230 and the driver 230 does not axially overlap with the friction brake 225 in between the friction brake 225 and the first contact section 211 of the nut 200.

The nut 200 forms a support member for the friction brake 225 and the friction brake is axially retained on the nut 200 by a friction fit. Furthermore, the driver 230 forms an outer member that at least partially surrounds the nut 200, which forms an inner member.

With alternative embodiments, the first contact section 211 may also be provided at the protruding part 220 of the nut 200. For example, the first contact section 211 may be formed at the distal surface of the protruding part 220. The friction brake 225 then may rest against the bars 221. In this case, the friction brake 225 is axially held in the proximal direction 5 at four separate locations around the circumference around the longitudinal axis 7.

Additionally or alternatively, the nut 200 may comprise an additional contact section that prevents the friction brake 225 from moving in the distal direction with respect to the nut 200. The additional contact section may be formed by a depression on the outer surface 206 of the nut 200. Additionally or alternatively, the additional contact section may comprise a radial protrusion that extends from the outer surface 206 and that overlaps with the friction brake 225 parallel to the longitudinal axis 7. The nut 200 then may axially overlap with the friction brake 225 in between the friction brake 225 and the second contact section 242 of the driver 230.

With further alternative embodiments, the friction brake 225 may also be configured as a disk-like washer that surrounds the nut 200. The washer may be provided at one of the first and second contact section 211, 242, for example at the first contact section 211.

Furthermore, the friction brake 225 may also be integrally formed with one of the first and second contact section 211, 242. For example, the friction brake 225 may be provided by one of the contact surfaces of the first and second contact sections 211, 242. The friction brake 225 then may be formed materially-uniform with the respective one of the first and second contact sections 211, 242.

The first contact section 211 may also be formed as a conical section of the outer surface 206 of the nut 200 and/or the second contact section 242 may also be formed as a conical section of the inner surface 246 of the driver 230, such as respective conical surfaces that are angled with respect to the radial plane perpendicular to the longitudinal axis 7.

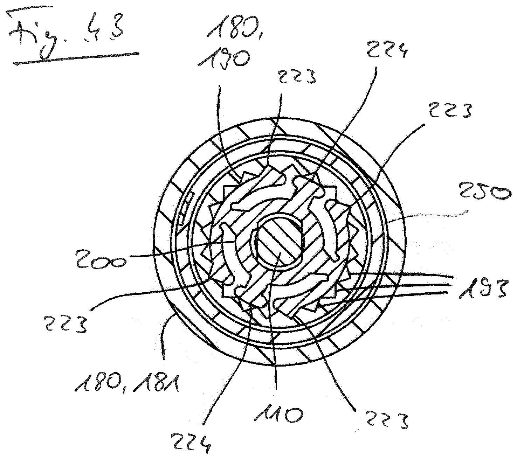

**Fig. 43** depicts a cross-sectional view of the driver 230, the nut 200, the inner housing 180, the dose member 250 and the piston rod 110 of the dosing mechanism 100 in a cut plane perpendicular to the longitudinal axis 7 through the protruding part 220 of the nut 200. The outer circumferential surface of the protruding part 220 rests against the longitudinal webs 193. Furthermore, the first latching parts 223 of the latching mechanism 192 engage with the longitudinal webs 193 and are positioned in between respective neighboring pairs of the longitudinal webs 193 to define a settable dose.

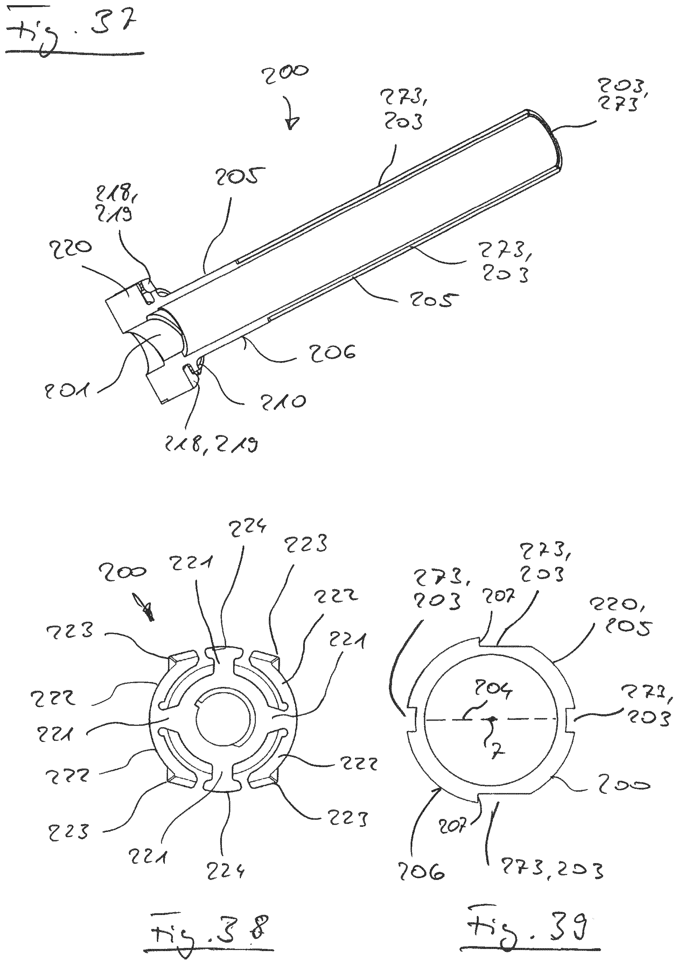

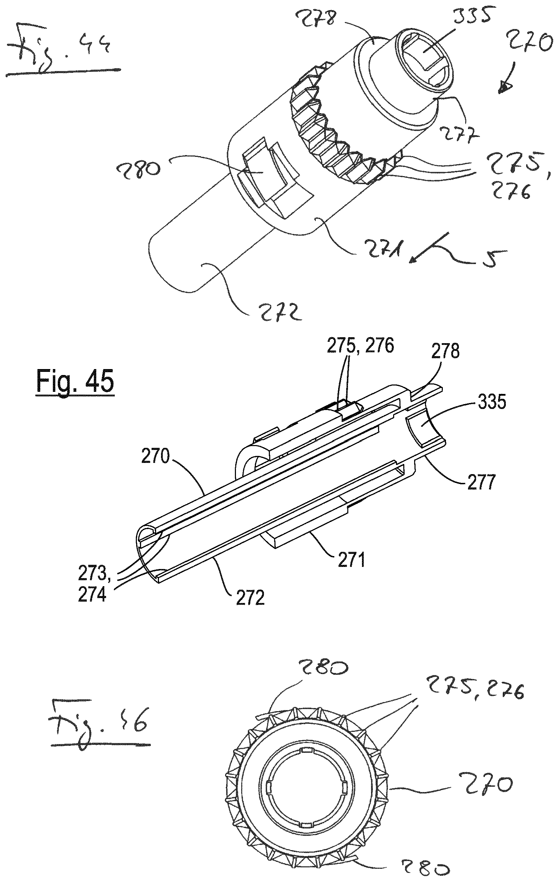

As can be seen from Fig. 39, the rotation lock 273 between the nut 200 and the connector 270 comprises four longitudinal depressions 203 that engage with corresponding longitudinal ridges 274 on an inside surface of the connector 270, which is shown in **Figs. 44** to **46****.** The longitudinal depressions 203 generally form first longitudinal elements of the rotation lock 273 that engage with corresponding second longitudinal elements of the rotation lock 273, which are formed by the longitudinal ridges 274.

The four longitudinal depressions 203 shown in Fig. 39 are located pairwise opposite each other in the radial direction and are equally spaced from each other around the circumference of the nut 200. Each depression 203 is configured to be fully accessible from one of two opposing radial directions, whereby the radial directions run horizontally in the view shown in Fig. 39. Each radial depression 203 is configured to form an undercut of the nut 200 only in at least one of the radial directions. As a consequence, each radial depression 203 is fully opened in one of the radial directions.

With the nut 200, the opposing depressions 203 are configured as rectangular depressions having radial side surfaces that run parallel to each other and parallel through a radial center axis 204, the radial center axis 204 cutting the central longitudinal axis 7 of the outer circumferential surface 206 of the nut 200. The remaining two opposing depressions 203 face each other in a direction that is perpendicular to the radial center axis 204. They each form a step within the outer surface 206 that has a radial side surface 207 running perpendicular to the radial center axis 204 and a bottom surface that runs parallel to the radial center axis 204 and extends from the step to the outer surface 206.

The depressions 203 being fully accessible from one of the two opposing radial directions allows to fabricate the nut 200 in an injection molding process with a mold that has two halves that are detachable by moving them apart parallel to the radial center axis 204 and that rest against each other at the radial side surfaces 207.

As can be seen from Figures 44 to 46, the longitudinal ridges 274 of the rotation lock 273 are provided at a central connection part 272 of the connector 270. The connection part 272 forms a tubular portion having a through hole. The connection part 272 is surrounded by a tubular clutch part 271 that comprises first clutch parts 276 of the clutch 275 on its outer surface. The first clutch parts 276 are configured as radially extending teeth.

The clutch part 271 is joined to the connection part 272 at the distal end of the clutch part 271, where it forms the shoulder 278 that rests against the bearing 360. The distal cylindrical part 277 extends distally from the shoulder 278 and has a reduced diameter compared to the clutch part 271. On the inside surface of the distal cylindrical part 277, the axial lock 335 is formed.

The dosing mechanism 100 comprises a feedback mechanism 280 that provides an audible and/or tactile feedback to a user during dose delivery. The feedback mechanism 280 comprises two flexible arms that protrude radially from the outer surface of the clutch part 271. The flexible arms are located opposite each other in the radial direction.

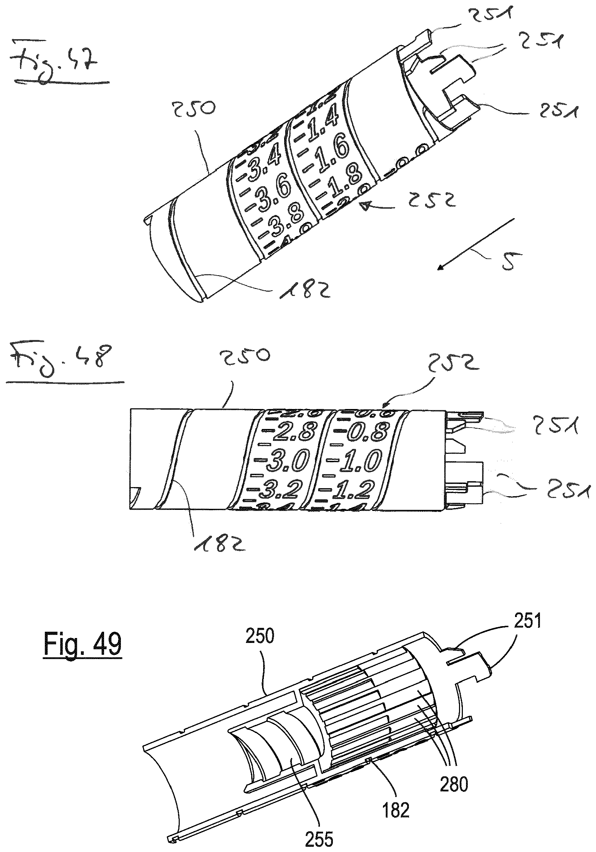

The flexible arms of the feedback mechanism 280 engage with radial teeth of the feedback mechanism 280 provided on an inside surface of the dose member 250, which is shown in **Figs. 47** to **49****.** The feedback mechanism 280 is active during dose delivery but not during dose setting since the connector 270 and the dose member 250 are rotationally fixed with each other during dose setting.

At a distal end of the dose member 250, a connector 251 is located that axially and rotationally fixes the dose member 252 the dose setting element 310. The connector 251 is configured as a snap-fit connector that allows to snap the dose setting element 310 onto the dose member 250 from the distal direction during assembly.

As can be seen from Fig. 49, an inner thread of the threaded connection 255 between the driver 230 and the dose member 250 is provided on an inside surface of the inner sleeve of the dose member 250. The inner sleeve is connected to an outer tubular portion of the dose member 250 by a shoulder. As can be seen from Figs. 23 to 25 the shoulder provides a bearing surface for the second biasing member 152. The inner thread of the threaded connection 255 extends over the entire length of the inner tubular portion.

The dose member 250 comprises the markings 252 on its outer surface that are visible through the window 164 in the housing 162 and that indicate the set dose. The markings 252 are arranged in a helical pattern on an outer surface of the dose member 250. The dose member 250 forms a dose indication member of the dosing mechanism 100.

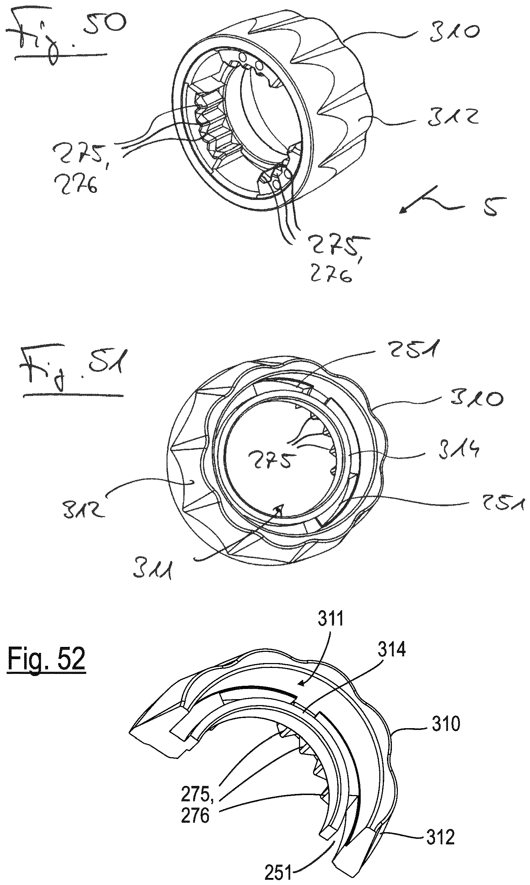

**Figs. 50** to **52** depict the dose setting element 310. The dose setting element 310 comprises the gripping structure 312 on its outer circumferential surface. The gripping structure 312 is longitudinally structured to provide an enhanced grip to a user of the device.

The dose setting element 310 has a central through hole that receives the distal cylindrical part 277 of the connector 270. At its distal end, the dose setting element 310 forms an open cavity 311 that receives the bearing 360 and the button ring 340. At a bottom of the cavity 311, the dose setting element 310 comprises a ring-like push surface 314 that rests against the bearing 360 when the button 330 is pushed in the proximal direction.

The push surface 314 is surrounded by openings of the connector 251 that axially and rotationally fix the dose setting element 310 to the dose member 250. The push surface 314 is thereby located at an end of a rim that extends from the openings in the distal direction within the cavity 311.

The dose setting element 310 further comprises a second clutch part 313 of the clutch 275. The second clutch part 313 is configured as radially extending teeth on an inside surface of the dose setting element 310. The second clutch part 313 is located at a proximal end of the dose setting element 310.

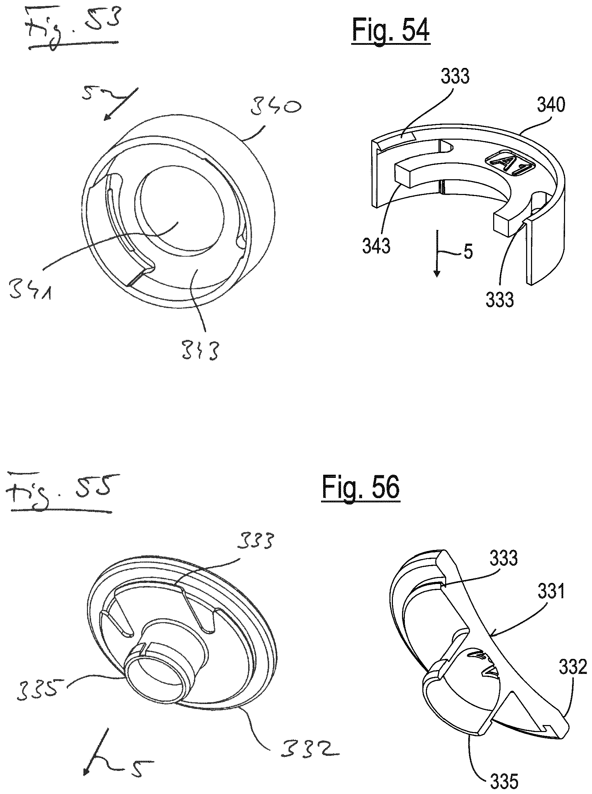

**Figs. 53** and **54** depict the button ring 340 and **Figs. 55** and **56** depict the button disc 332. The button ring 340 has an inner radial wall and a proximal surface of the inner radial wall forms a push surface 343 that rests against the distal end surface of the bearing 360.

A central through hole 341 in the inner radial wall receives a tubular portion of the axial lock 335, which tubular portion is provided at a proximal side of the button disc 332. The tubular portion has radially extending ridges on its outer circumferential surface which engage with corresponding radial protrusions on an inside surface of the distal cylindrical part 277 of the connector 270. The tubular portion of the button disc 332 is thereby received within the distal cylindrical part 277.

The axial lock 333 between the button disc 332 and the button ring 340 is formed by inwardly protruding radial protrusion at a distal end of the button ring 340 and corresponding radially protruding members provided at a proximal surface of the button disc 332. The axial lock 333 is configured as a snap-fit connection that allows to snap the button disc 332 to distal end of the button ring 340. A distal end surface of the button disc 332 forms a push surface 331 that is configured to be actuated by the user push the button 330 in the proximal direction 5.

Alternative embodiments of the medicament delivery device 1 may only comprise one, two or three of the cap alignment mechanism 80, the blocking mechanism 70, the friction brake 225 and the hard stop 218.

Furthermore, the medicament delivery device 1 is only one exemplary medicament delivery device that may feature the cap alignment mechanism 80, the blocking mechanism 70, the friction brake 225 and the hard stop 218. For example, the alignment mechanism 80 and/or the friction brake 225 and/or the hard stop 118 may also be provided with a medicament delivery device that is configured as a disposable device with a medicament holder non-releasably attached to a housing. In addition, the friction brake 225 and/or the hard stop 118 may be provided with any medicament delivery device that has a first member that is configured to rotate with respect to a second member around a longitudinal axis during dose setting and that is configured to transfer an axial force to the second member in the proximal direction during dose delivery.

The present disclosure is, inter alia, directed at the following enumerated embodiments:
1. Dosing mechanism (100) for a medicament delivery device (1),
   the dosing mechanism (100) comprising a first member (230), a second member (200) and a friction brake (225),
   wherein the friction brake (225) is located in between a first contact section (211) of the first member (230) and a second contact section (242) of the second member (200),
   wherein the first member (230) is configured to rotate with respect to the second member (200) around a longitudinal axis (7) during setting of a dose of medicament to be delivered,
   wherein the first member (230) is configured to transfer an axial force to the second member (200) in a proximal direction (5) parallel to the longitudinal axis (7) during dose delivery to deliver the dose,
   wherein the first contact section (211) is configured to press the friction brake (225) towards the second contact section (242) during dose delivery so that the friction brake (225) provides a rotation brake between the first member (230) and the second member (200).
2. The dosing mechanism (100) of embodiment 1,
   wherein the dosing mechanism (100) comprises a hard stop (218) that acts between the first member (230) and the second member (200) during dose delivery,
   wherein the hard stop (218) is configured to restrict a movement of the first contact section (211) towards the second contact section (242) during dose delivery and to limit the pressure applied to the friction brake (225).
3. The dosing mechanism (100) of embodiment 2,
   wherein the axial force is transferred from the first member (230) to the second member (200) via the hard stop (218) while the hard stop (218) is restricting the movement.
4. The dosing mechanism (100) of at least one of embodiments 2 and 3,
   wherein the hard stop (218) has a first stop part (244) axially fixed to the first member (230) and a second stop part (219) axially fixed to the second member (200),
   wherein engagement of the first stop part (244) with the second stop part (219) during dose delivery restricts the movement of the first contact section (211) with respect to the second contact section (242).
5. The dosing mechanism (100) of at least embodiment 4,
   wherein the first stop part (244) is integrally formed with the first member (230) and/or the second stop part (219) is integrally formed with the second member (200).
6. The dosing mechanism (100) of at least one of embodiments 4 to 5,
   wherein at least one of the first stop part (244) and the second stop part (219) circumferentially surrounds the longitudinal axis (7).
7. The dosing mechanism (100) of at least one of embodiments 2 to 6,
   wherein the hard stop (218) is configured as an axial stop,
   wherein, for example, the first stop part (244) is configured to move against the second stop part (219) parallel to the longitudinal axis (7) upon engagement of the hard stop (218).
8. The dosing mechanism (100) of at least one of embodiments 2 to 7,
   wherein the hard stop (218) has a first stop surface axially fixed to the first member (230) and a second stop surface axially fixed to the second member (200),
   wherein the first stop surface and the second stop surface are orientated perpendicular to the longitudinal axis (7).
9. The dosing mechanism (100) of at least one of embodiments 2 to 8,
   wherein the hard stop (218) defines a maximal compression of the friction brake (225),
   wherein, for example, the maximal compression is at most 0.2 mm, such as at most 0.15 mm, at most 0.1 mm, at most 0.9 mm, at most 0.8 mm or at most 0.7 mm.
10. The dosing mechanism (100) of at least one of the preceding embodiments,
   wherein the first contact section (211) circumferentially extends around the longitudinal axis (7), and/or
   wherein the second contact section (242) circumferentially extends around the longitudinal axis (7).
11. The dosing mechanism (100) of at least one of the preceding embodiments,
   wherein the first contact section (211) is configured as a surface that is perpendicular to the longitudinal axis (7), and/or
   wherein the second contact section (242) is configured as a surface that is perpendicular to the longitudinal axis (7).
12. The dosing mechanism (100) of at least one of the preceding embodiments,
   wherein the first contact section (211) is configured as a first surface and the second contact section (242) is configured as a second surface,
   wherein the first surface is parallel to the second surface.
13. The dosing mechanism (100) of at least one of the preceding embodiments,
   wherein the friction brake (225) is configured as a separate friction member that is placed in between the first contact section (211) and the second contact section (242).
14. The dosing mechanism (100) of at least embodiment 13 and at least one of embodiments 2 to 9,
   wherein the hard stop (218) defines a gap between the first contact section (211) and the second contact section (242) during dose delivery,
   wherein the gap accommodates the friction brake (225),
   wherein, for example, the gap is an axial gap.
15. The dosing mechanism (100) of at least one of embodiments 13 and 14,
   wherein the first member (230) does not axially overlap with the friction brake (225) in between the friction brake (225) and the second contact section (242) of the second member (200), and/or wherein the second member (200) does not axially overlap with the friction brake (225) in between the friction brake (225) and the first contact section (211) of the first member (230).
16. The dosing mechanism (100) of at least one of embodiments 13 to 15,
   wherein the friction brake (225) is configured as a ring-like structure, such as a torus or a washer.
17. The dosing mechanism (100) of at least one of embodiments 13 to 16,
   wherein one of the first member (230) and the second member (200) forms a support member, wherein the friction brake (225) is located on an outer surface (206) of the support member.
18. The dosing mechanism (100) of at least embodiment 17,
   wherein the friction brake (225) is axially retained on the support member, for example by a friction fit.
19. The dosing mechanism (100) of at least one of embodiments 13 to 18,
   wherein the friction brake (225) is located in a cavity (226) formed between the first member (230) and the second member (200),
   wherein the cavity (226) is radially bounded by the first member (230) and the second member (200),
   wherein the cavity (226) is axially bounded by a first contact structure (210) comprising the first contact section (211) and a second contact structure (243) comprising the second contact section (242),
   wherein a first radial spacing (615) between the first contact structure (210) and the second member (200) is smaller than a radial height (227) of the friction brake (225), and/or wherein a second radial spacing (616) between the second contact structure (243) and the first member (230) is smaller than the radial height (227) of the friction brake (225).
20. The dosing mechanism (100) of at least one of the preceding embodiments,
   wherein the friction brake (225) is configured as an elastic member,
   wherein, for example, the friction brake (225) comprises rubber or consists of rubber.
21. The dosing mechanism (100) of at least one of the preceding embodiments,
   wherein a hardness of the friction brake (225) is lower than a hardness of the first contact section (211) and/or a hardness of the second contact section (242).
22. The dosing mechanism (100) of at least one of the preceding embodiments,
   wherein the friction brake (225) has at most a Shore A hardness of 90, such as at most a Shore A hardness of 85, at most a Shore A hardness of 80, or at most a Shore A hardness of 75.
23. The dosing mechanism (100) of at least one of the preceding embodiments,
   wherein one of the first member (230) and the second member (200) forms an inner member and the other one of the first member (230) and the second member (200) forms an outer member,
   wherein the outer member at least partly surrounds the inner member.
24. The dosing mechanism (100) of at least embodiment 23,
   wherein the outer member radially overlaps with the friction brake (225),
   wherein, for example, the friction brake (225) is located in an end cavity (239) of the outer member.
25. The dosing mechanism (100) of at least embodiment 24,
   wherein the outer member is radially spaced from the friction brake (225) by a clearance (613).
26. The dosing mechanism (100) of at least embodiment 25,
   wherein the clearance (613) is smaller than the radial height (227) of the friction brake (225),
   wherein, for example, the clearance (613) is at most 0.5 times, such as at most 0.4 times, or at most 0.3 times a radial height (227) of the friction brake (225).
27. The dosing mechanism (100) of at least one of embodiments 23 to 26 and at least one of embodiments 17 to 19,
   wherein the inner member forms the support member.
28. The dosing mechanism (100) of at least one of the preceding embodiments,
   wherein the first contact section (211) axially overlaps with the friction brake (225) over a first radial height (610) that is at least 0.5 times, such as at least 0.6 times, at least 0.7 times or at least 0.8 times a radial height (227) of the friction brake (225) and/or
   wherein the second contact section (242) axially overlaps with the friction brake (225) over a second radial height (611) that is at least 0.5 times, such as at least 0.6 times, at least 0.7 times or at least 0.8 times a radial height (227) of the friction brake (225).
29. The dosing mechanism (100) of at least one of the preceding embodiments,
   wherein the dosing mechanism (100) comprises a further stop (208),
   wherein the further stop (208) acts between the first member (230) and the second member (200),
   wherein the further stop (208) restrains axial movement of the first contact section (211) away from the second contact section (242) during dose setting.
30. The dosing mechanism (100) of at least one of the preceding embodiments,
   wherein one of the first member (230) and the second member (200) is permanently rotationally fixed to a housing (160) of the dosing mechanism (100).
31. The dosing mechanism (100) of at least one of the preceding embodiments,
   wherein the dosing mechanism (100) comprises a latching mechanism (192) that defines discrete rotational positions of the second member (200) with respect to the first member (230), wherein the first member (230) is permanently rotationally fixed to a first latching part (223) of the latching mechanism (192),
   wherein the second member (200) is permanently rotationally fixed to a second latching part (193) of the latching mechanism (192),
   wherein the first latching part (223) and the second latching part (193) releasably engage with each other upon relative rotation of the first member (230) relative to the second member (200), wherein the latching mechanism (192) provides an additional rotation brake preventing relative rotation of the first member (230) and the second member (200) during dose delivery.
32. The dosing mechanism (100) of at least embodiment 31,
   wherein the second latching part (193) is axially fixed to the second member (200),
   wherein the second contact section (242) and the second latching part (193) are separate from each other.
33. The dosing mechanism (100) of at least one of the preceding embodiments,
   wherein the dosing mechanism (100) comprises a piston rod (110) that is configured to move into a medicament holder (40) attachable to the dosing mechanism (100) to deliver the dose from the medicament holder (40),
   wherein the piston rod (110) is rotationally fixed with respect to the first member (230) at least during dose delivery,
   wherein the second member (200) is coupled to the piston rod (110), for example engages the piston rod (110), via a threaded connection (112).
34. A medicament delivery device (1) having the dosing mechanism (100) of at least one of the preceding embodiments.

The present disclosure is also directed at the following embodiments of a medicament delivery device:
1. A medicament delivery device (1) having
   a medicament holder (40), a housing (160), and a cap (10),
   wherein the medicament holder (40) is configured to releasably attach to the housing (160) via a connector by rotating the medicament holder (40) around a longitudinal axis (7) with respect to the housing (160),
   wherein the cap (10) is configured to releasably attach to the medicament holder (40) to at least partly cover the medicament holder (40).
2. The medicament delivery device (1) of embodiment 1,
   wherein the medicament holder (40) comprises a first blocking element (50) of a blocking mechanism (70) that is configured to engage with a second blocking element (167) of the blocking mechanism (70), the second blocking element (167) being provided at the housing (160),
   wherein the blocking mechanism (70) is interlocked by the cap (10) to rotationally lock the medicament holder (40) to the housing (160) when the cap (10) is attached to the medicament holder (40),
   wherein the blocking mechanism (70) is unlocked to allow rotation of the medicament holder (40) with respect to the housing (160) when the cap (10) is detached from the medicament holder (40).
3. The medicament delivery device (1) of embodiment 2,
   wherein one of the first blocking element (50) and the second blocking element (167) is attached to a flexible element (52) that is configured to bend away from the other one of the first blocking element (50) and the second blocking element (167) to allow the rotation of the medicament holder (40) with respect to the housing (160),
   wherein the attachment of the cap (10) to the medicament holder (40) blocks the flexible element (52) from bending.
4. The medicament delivery device (1) of embodiment 3,
   wherein the cap (10) is configured to engage with the flexible element (52) to block the flexible element (52) from bending.
5. The medicament delivery device (1) of at least one of embodiments 3 and 4,
   wherein the flexible element (52) is configured to bend radially outwards to allow the rotation of the medicament holder (40) with respect to the housing (160).
6. The medicament delivery device (1) of at least one of embodiments 3 to 5,
   wherein the flexible element (52) is provided at the medicament holder (40).
7. The medicament delivery device (1) of at least embodiment 6,
   wherein the flexible element (52) is integrally formed with the medicament holder (40).
8. The medicament delivery device (1) of at least embodiments 6 and 7,
   wherein the flexible element (52) is accessible at an outer surface (45) of the medicament holder (40).
9. The medicament delivery device (1) of at least one of embodiments 3 to 8
   wherein the flexible element (52) has a fixed first end (51) and a free second end (53),
   wherein the one of the first and second blocking element (50, 167) is attached to the second end (53),
   wherein the first end (51) is located opposite the second end (53) along the longitudinal axis (7).
10. The medicament delivery device (1) of at least one of embodiments 2 to 9,
   wherein the blocking mechanism (70) is configured as a radial block,
   wherein the first blocking element (50) is configured to engage with the second blocking element (167) by rotating with respect to the second blocking element (167) around the longitudinal axis (7).
11. The medicament delivery device (1) of at least one of embodiments 2 to 10,
   wherein one of the first blocking element (50) and the second blocking element (167) is configured as a radial protrusion and the other one of the first blocking element (50) and the second blocking element (167) is configured as a radial detent that is configured to receive the radial protrusion.
12. The medicament delivery device (1) of at least embodiment 11,
   wherein the radial detent has a larger length in a circumferential direction (6) than the radial protrusion.
13. The medicament delivery device (1) of at least one of embodiments 11 and 12,
   wherein the radial detent has a first side surface (166) in a first circumferential direction and a second side surface (168) in a second circumferential direction (6) opposite the first circumferential direction,
   wherein the first side surface (166) is engaged by the radial protrusion upon rotating the medicament holder (40) to detach the medicament holder (40) from the housing (160),
   wherein the first side surface (166) is steeper than the second side surface (168).
14. The medicament delivery device (1) of at least of the preceding embodiments,
   wherein the connector (90) comprises a first connection element (41) provided at the medicament holder (40),
   wherein the first connection element (41) is configured as a female connector that is configured to receive a second connection element (163) provided at the housing (160).
15. The medicament delivery device (1) of at least one of the preceding embodiments,
   wherein the connector (90) is configured as a threaded connector.
16. The medicament delivery device (1) of at least one of the preceding embodiments,
   wherein the medicament holder (40) comprises a first cap lock element (47) that is configured to engage with a second cap lock element (21) of the cap (10) to axially hold the cap (10) at the medicament holder (40).
17. The medicament delivery device (1) of at least embodiment 16,
   wherein a cap lock (85) formed by the first cap lock element (47) and the second cap lock element (21) is configured as an axial lock that does not restrict rotation of the cap (10) with respect to the medicament holder (40).
18. The medicament delivery device (1) of at least one of the preceding embodiments,
   wherein the medicament holder (40) comprises a first aligning part (60) of a cap alignment mechanism (80) and the cap comprises a second aligning part (35) of the cap alignment mechanism (80),
   wherein the first aligning part (60) is configured to engage with the second aligning part (35) upon attachment of the cap (10) to the medicament holder (40) to attach the cap (10) in a predefined rotational orientation to the housing (160).
19. The medicament delivery device (1) of at least embodiment 18,
   wherein the first aligning part (60) and/or the second aligning part (35) comprise a lead-in taper that narrows in a distal direction parallel to a longitudinal axis (7).
20. The medicament delivery device (1) of at least embodiment 19,
   wherein both the first aligning part (60) and the second aligning part (35) comprise the lead-in taper,
   wherein a first taper angle (81) of the first aligning part (60) is larger than a second taper angle (82) of the second aligning part (35).
21. The medicament delivery device (1) of at least one of embodiments 19 and 20 ,
   wherein the lead-in taper is configured as a non-self-locking taper,
   wherein the lead-in taper forces the cap (10) to move in a proximal direction (5) parallel to the longitudinal axis (7) upon rotating the cap (10) while being fully attached to the housing (160).
22. The medicament delivery device (1) of at least one of embodiments 18 to 21,
   wherein one of the first aligning part (60) and the second aligning part (35) is configured as a radial protrusion and the other one of the first aligning part (60) and the second aligning part (35) is configured as a radial depression that receives the radial protrusion upon attachment of the cap to the housing (160).
23. The medicament delivery device (1) of at least embodiment 22,
   wherein in the radial depression has an open proximal end.
24. The medicament delivery device (1) of at least one of embodiments 22 and 23,
   wherein the radial depression comprises a first radial sidewall (64) and the radial protrusion comprises a second radial sidewall (37),
   wherein the first radial sidewall (64) and/or the second radial sidewall (37) are angled with respect to a radial plane perpendicular to the longitudinal axis (7),
   wherein the first radial sidewall (64) is configured to slide along the second radial sidewall (37) upon attachment of the cap (10) to the housing (160) to align the cap (10) in the predefined rotational orientation.
25. The medicament delivery device (1) of at least embodiment 24,
   wherein the first radial sidewall (64) and/or the second radial sidewall (37) are angled with respect to the longitudinal axis (7).
26. The medicament delivery device (1) of at least one of embodiments 24 and 25,
   wherein the first radial sidewall (64) and the second radial sidewall (37) comprise an undercut in a cross-sectional plane perpendicular to the longitudinal axis (7).
27. The medicament delivery device (1) of at least one of embodiments 18 to 26,
   wherein the cap (10) comprises a first member (30) comprising the second aligning part (35) and a second member (20) non-releasably joined to the first member (30), for example by an adhesive bond,
   wherein, for example, the first member (30) is glued to the second member (20).
28. The medicament delivery device (1) of at least embodiment 27,
   wherein the first member (30) comprises a further first aligning part (34) of a body alignment mechanism (33) and the second member (20) comprises a further second aligning part (23) of the body alignment mechanism (33),
   wherein the engagement of the further first aligning part (34) with the further second aligning part (23) is configured to rotationally align the first member (30) with respect to the second member (20).
29. The medicament delivery device (1) of at least one of embodiments 27 and 28,
   wherein the first member (30) forms an inner part and the second member (20) forms an outer part at least partly surrounding the inner part.
30. The medicament delivery device (1) of at least one of embodiments 27 to 29,
   wherein the first member (30) comprises a plastic material, for example consists of the plastic material,
   wherein the second member (20) comprises a metal material, for example consists of the metal material.
31. The medicament delivery device (1) of at least one of embodiments 18 to 30,
   wherein the cap (10) comprises a cap body (20) and a cap end element (30),
   wherein the cap end element (30) is located at a proximal end of the cap body (20),
   wherein the second aligning part (35) is formed at the cap end element (30).
32. The medicament delivery device (1) of at least one of embodiments 18 to 31,
   wherein the first aligning part (60) is made from a plastic material, such as a thermoplastic material, and/or
   wherein the second aligning part (35) is made from a plastic material, such as a thermoplastic material.
33. The medicament delivery device (1) of at least one of embodiments 18 to 32,
   wherein the first aligning part (60) is configured as a molded part, such as an injection molded part, and/or
   wherein the second aligning part (35) is configured as a molded part, such as an injection molded part.
34. The medicament delivery device (1) of at least one of embodiments 18 to 33,
   wherein the first aligning part (60) is located at a proximal end of the medicament holder (40) and/or
   wherein the second aligning part (35) is located at a proximal end of the cap (10).

The present disclosure also relates to a medicament holder having the features of the medicament holder of the medicament delivery device of at least one of the preceding embodiments. Furthermore, the present disclosure relates to a housing having the features of the housing of the medicament delivery device of at least one of the preceding embodiments.

### List of reference signs

- 1: medicament delivery device
- 2: proximal end
- 3: distal end
- 5: proximal direction
- 6: circumferential direction
- 7: longitudinal axis
- 10: cap
- 20: cap body
- 21: second cap lock element
- 23: second aligning part
- 25: proximally facing surface
- 28: distally facing surface
- 29: recessed section
- 30: cap end element
- 31: centering element
- 32: protruding part
- 33: body alignment mechanism
- 34: first aligning part
- 35: second aligning part
- 36: surface
- 37: second radial sidewall
- 38: outer part
- 39: inner part
- 40: medicament holder
- 41: first connection element
- 42: needle connector
- 43: protruding axial section
- 44: window
- 45: outer surface
- 46: step
- 47: first cap lock element
- 48: recessed axial section
- 50: first blocking element
- 51: first end
- 52: flexible element
- 53: second end
- 54: gap
- 56: flexing portion
- 60: first aligning part
- 64: first radial sidewall
- 70: blocking mechanism
- 80: cap alignment mechanism
- 81: first taper angle
- 82: second taper angle
- 85: cap lock
- 90: connector
- 95: axial stop
- 100: dosing mechanism
- 110: piston rod
- 112: threaded connection
- 114: last dose stop
- 120: piston bearing
- 122: connector
- 130: piston rod guide
- 132: gripping structure
- 133: second stop member
- 134: through hole
- 135: second lock member
- 150: first biasing member
- 152: second biasing member
- 160: housing
- 162: outer housing
- 163: second connection element
- 164: window
- 165: axial lock
- 166: first side surface
- 167: second blocking element
- 168: second side surface
- 170: end surface
- 171: recess
- 172: recess
- 180: inner housing
- 181: outer section
- 182: threaded connection
- 183: proximal end surface
- 184: protrusion
- 185: protrusion
- 186: first stop member
- 188: first lock member
- 190: inner section
- 192: latching mechanism
- 193: second latching part (longitudinal web)
- 200: nut
- 201: inner thread
- 202: rotation lock
- 203: depression
- 204: center axis
- 205: body
- 206: outer surface
- 207: radial side surface
- 208: further stop
- 209: first stop element
- 210: first contact structure
- 211: first contact section (contact surface)
- 218: hard stop
- 219: second stop part (contact surface)
- 220: protruding part
- 221: bar
- 222: flexible arm
- 223: first latching part (longitudinal element)
- 224: rigid arm
- 225: friction brake
- 226: cavity
- 227: radial height
- 230: driver
- 231: outer thread
- 232: body
- 235: end stop
- 237: second stop element
- 239: end cavity
- 240: protruding part
- 241: lock element
- 242: second contact section (contact surface)
- 243: second contact structure
- 244: first stop part (contact surface)
- 246: inner surface
- 250: dose member
- 251: connector
- 252: marking
- 255: threaded connection
- 270: connector
- 271: clutch part
- 272: connection part
- 273: rotation lock
- 274: ridge
- 275: clutch
- 276: first clutch part
- 277: distal cylindrical part
- 278: shoulder
- 280: feedback mechanism
- 300: actuation unit
- 310: dose setting element
- 311: cavity
- 312: gripping structure
- 313: second clutch part
- 314: push surface
- 330: button
- 331: push surface
- 332: button disc
- 333: axial lock
- 335: axial lock
- 340: button ring
- 341: through hole
- 343: push surface
- 360: bearing
- 500: medicament container
- 502: container body
- 503: medicament compartment
- 504: septum
- 510: piston
- 601: axial distance
- 603: axial distance
- 605: axial distance
- 610: first radial height
- 611: second radial height
- 613: clearance
- 615: first radial spacing
- 616: second radial spacing

## Claims

1. Dosing mechanism (100) for a medicament delivery device (1),
the dosing mechanism (100) comprising a first member (230), a second member (200) and a friction brake (225),
wherein the friction brake (225) is located in between a first contact section (211) of the first member (230) and a second contact section (242) of the second member (200),
wherein the first member (230) is configured to rotate with respect to the second member (200) around a longitudinal axis (7) during setting of a dose of medicament to be delivered, wherein the first member (230) is configured to transfer an axial force to the second member (200) in a proximal direction (5) parallel to the longitudinal axis (7) during dose delivery to deliver the dose,
wherein the first contact section (211) is configured to press the friction brake (225) towards the second contact section (242) during dose delivery so that the friction brake (225) provides a rotation brake between the first member (230) and the second member (200),
wherein the dosing mechanism (100) comprises a hard stop (218) that acts between the first member (230) and the second member (200) during dose delivery,
wherein the hard stop (218) is configured to restrict a movement of the first contact section (211) towards the second contact section (242) during dose delivery and to limit the pressure applied to the friction brake (225).

2. The dosing mechanism (100) of claim 1,
wherein the axial force is transferred from the first member (230) to the second member (200) via the hard stop (218) while the hard stop (218) is restricting the movement.

3. The dosing mechanism (100) of at least one of the preceding claims,
wherein the hard stop (218) is configured as an axial stop,
wherein, for example, a first stop part (244) axially fixed to the first member (230) is configured to move against a second stop part (219) axially fixed to the second member (200) parallel to the longitudinal axis (7) upon engagement of the hard stop (218).

4. The dosing mechanism (100) of at least one of the preceding claims,
wherein the hard stop (218) defines a maximal compression of the friction brake (225), wherein, for example, the maximal compression is at most 0.2 mm, such as at most 0.15 mm, at most 0.1 mm, at most 0.9 mm, at most 0.8 mm or at most 0.7 mm.

5. The dosing mechanism (100) of at least one of the preceding claims,
wherein the friction brake (225) is configured as a separate friction member that is placed in between the first contact section (211) and the second contact section (242).

6. The dosing mechanism (100) of at least claim 5,
wherein the hard stop (218) defines a gap between the first contact section (211) and the second contact section (242) during dose delivery,
wherein the gap accommodates the friction brake (225),
wherein, for example, the gap is an axial gap.

7. The dosing mechanism (100) of at least one of claims 5 and 6,
wherein the first member (230) does not axially overlap with the friction brake (225) in between the friction brake (225) and the second contact section (242) of the second member (200), and/or
wherein the second member (200) does not axially overlap with the friction brake (225) in between the friction brake (225) and the first contact section (211) of the first member (230).

8. The dosing mechanism (100) of at least one of claims 5 to 7,
wherein the friction brake (225) is configured as a ring-like structure, such as a torus or a washer.

9. The dosing mechanism (100) of at least one of claims 5 to 8,
wherein the friction brake (225) is located in a cavity (226) formed between the first member (230) and the second member (200),
wherein the cavity (226) is radially bounded by the first member (230) and the second member (200),
wherein the cavity (226) is axially bounded by a first contact structure (210) comprising the first contact section (211) and a second contact structure (243) comprising the second contact section (242),
wherein a first radial spacing (615) between the first contact structure (210) and the second member (230) is smaller than a radial height (227) of the friction brake (225), and/or wherein a second radial spacing (616) between the second contact structure (243) and the first member (230) is smaller than the radial height (227) of the friction brake (225).

10. The dosing mechanism (100) of at least one of the preceding claims,
wherein the friction brake (225) is configured as an elastic member,
wherein, for example, the friction brake (225) comprises rubber or consists of rubber.

11. The dosing mechanism (100) of at least one of the preceding claims,
wherein one of the first member (230) and the second member (200) forms an inner member and the other one of the first member (230) and the second member (200) forms an outer member, wherein the outer member at least partly surrounds the inner member,
wherein the outer member radially overlaps with the friction brake (225),
wherein, for example, the friction brake (225) is located in an end cavity (239) of the outer member.

12. The sing mechanism (100) of at least claim 11,
wherein the outer member is radially spaced from the friction brake (225) by a clearance (613), wherein the clearance (613) is smaller than the radial height (227) of the friction brake (225),
wherein, for example, the clearance (613) is at most 0.5 times, such as at most 0.4 times, or at most 0.3 times a radial height (227) of the friction brake (225).

13. The dosing mechanism (100) of at least one of the preceding claims,
wherein the first contact section (211) axially overlaps with the friction brake (225) over a first radial height (610) that is at least 0.5 times, such as at least 0.6 times, at least 0.7 times or at least 0.8 times a radial height (227) of the friction brake (225) and/or
wherein the second contact section (242) axially overlaps with the friction brake (225) over a second radial height (611) that is at least 0.5 times, such as at least 0.6 times, at least 0.7 times or at least 0.8 times a radial height (227) of the friction brake (225).

14. The dosing mechanism (100) of at least one of the preceding claims,
wherein the dosing mechanism (100) comprises a latching mechanism (192) that defines discrete rotational positions of the second member (230) with respect to the first member (200), the first member (230) is permanently rotationally fixed to a first latching part (223) of the latching mechanism (192),
wherein the second member (200) is permanently rotationally fixed to a second latching part (193) of the latching mechanism (192),
wherein the first latching part (223) and the second latching part (193) releasably engage with each other upon relative rotation of the first member (230) relative to the second member (200), wherein the latching mechanism (192) provides an additional rotation brake preventing relative rotation of the first member (230) and the second member (200) during dose delivery.

15. The dosing mechanism (100) of at least claim 14,
wherein the second latching part (193) is axially fixed to the second member (200),
wherein the second contact section (242) and the second latching part (193) are separate from each other.
